# EUROPEAN PATENT APPLICATION

(11) **EP 2 283 845 A1**
(43) Date of publication of application: **16.02.2011**
(21) Application number: 10185074.1
(22) Date of filing: 26.04.2002
(51) Int. Cl.: A61K 31/513, A61L 31/16, A61P 35/00

(54) **Sustained release drug delivery system containing codrugs**

(30) Priority: 26.04.2001 US 286343 P; 17.09.2001 US 322428 P; 15.04.2002 US 372761 P
(62) Divisional of application: 02729028.7
(71) Applicant: pSivida Inc., Watertown, MA 02472 (US)
(72) Inventor: Chen, Jianbing, Belmont, MA 02478-4628 (US); Ashton, Paul, Boston, MA MA 02108-1025 (US); Smith, Thomas, Weston, MA 02493-2176 (US)
(74) Representative: Jones, Helen M.M.

(57) **Abstract**

Disclosed is a sustained release system that includes a polymer and a prodrug having a solubility less than about 1 mg/ml dispersed in the polymer. Advantageously, the polymer is permeable to the prodrug and may be non-release rate limiting with respect to the rate of release of the prodrug from the polymer. This permits improved drug delivery within a body in the vicinity of a surgery via sustained release rate kinetics over a prolonged period of time, while not requiring complicated manufacturing processes.

## Description

### Field Of The Invention

The present invention relates generally to an improved system of delivering drugs. In particular, the present invention relates to a polymer-based, sustained-release drug delivery system and methods of delivering drugs using the same.

### Background Of The Invention

The desirability of sustained release has long been recognized in the pharmaceutical field. Many polymer-based systems have been proposed to accomplish the goal of sustained release. These systems generally have relied upon either degradation of the polymer or diffusion through the polymer as a means to control release.

Implantable drug delivery devices offer an attractive alternative to oral, parenteral, suppository, and topical modes of administration. For example, as compared to oral, parenteral and suppository modes of administration, implantable drug delivery permits more localized administration of drug than do other modes of administration. Thus, implantable drug delivery devices are especially desirable where a clinician wishes to elicit a more localized therapeutic pharmaceutical effect. Additionally, the ability of implantable drug delivery devices to deliver the drug directly to the desired site of action permits the clinician to use drugs that are relatively poorly absorbed, or labile in biological fluids, often to great advantage. Implantable drug delivery devices allow achievement of therapeutic doses at the desired site of action, while maintaining low or negligible systemic levels. Thus implantable drug delivery devices are especially attractive in situations where the drugs in question are toxic or have poor clearance characteristics, or both.

As compared to topical modes of administration, implantable drug delivery devices have the advantage that they can be applied subcutaneously. They can be surgically implanted and thereby deliver drug locally and in high concentrations over a protracted period of time. In comparison, topical application of drugs generally is limited to the epidermis, and must be repeated periodically to maintain concentration of the drug in its therapeutically effective range. Delivery by a transdermal route, such as by a transdermal patch, has the disadvantage of delivering drug systemically.

Despite the obvious advantages of implantable drug delivery devices, there are several needs left to be satisfied by implantable devices. For instance, there is a need for a simple drug delivery device that releases drug at a constant rate. Prior art attempts to solve this problem have met with limited success because they were difficult to construct and inconvenient to use.

There is therefore a need for an improved drug delivery device that provides sustained-release drug delivery within a body over a prolonged period of time that does not require complicated manufacturing processes.

Modem surgical methods employ various and numerous devices that are routinely placed within the body and left there for extended periods of time. Such devices include, but are not limited to sutures, stents, surgical screws, prosthetic joints, artificial valves, plates, pacemakers, etc. Such devices have proven useful over time, however some problems associated with implanted surgical devices remain. For instance, stents, artificial valves, and to some extent even sutures may be associated with restenosis after vascular surgery. It is therefore often necessary to use systemic drugs in conjunction with implantation of surgical devices, which increases the risk of post-operative hemorrhage. Occasionally, surgical implants may be subject to immune response or rejection. Consequently, it is sometimes necessary to abandon surgical implant therapy, or to use immune suppressant drugs in conjunction with certain surgical implants. In an effort to avoid systemic treatment the use of drugs in rate controlling bioerodible polymers has been frequently reported. Such systems are designed to release drug as the polymer erodes. This severely limits the selection of drug and polymer.

There is therefore a need for an improved drug delivery device that is capable of delivering a drug having anti-restenosis or immune suppressive activity in the vicinity of a surgical implant over a prolonged period at a sustained concentration within the therapeutically effective concentration range for the drug.

Many advances have been made to reduce the exposure of patients to pathogenic microbes during surgery, implantation of surgical devices nonetheless involves introducing into the body a foreign object that has the potential to infect patients with various viruses and/or bacteria. Accordingly, surgical procedures often result in infections to which a patient would not ordinarily be exposed, and which may compromise or negate the effectiveness of implantation therapy. Administration of antibiotics, corticosteroids and/or antivirals is therefore a common adjunct to implantation therapy, either for prophylaxis or in response to infection. However, systemic administration of such antimicrobial compositions often leads to undesirable side effects.

There is therefore a need for an improved drug delivery device that is capable of delivering a drug having antimicrobial activity in the vicinity of a surgical implant over a prolonged period at a sustained concentration within the therapeutically effective concentration range for the drug.

Surgical implantation often leads to other deleterious side effects such as pain and swelling. It is routine to treat surgical implant patients with anti-inflammatory and analgesic drugs, such as steroidal anti-inflammatories, non-steroidal anti-inflammatories (NSAIDs), such as aspirin, cefacoxib, rofecoxib, or indomethacin, other analgesics, such as acetaminophen, and opiates. As some post operative patients experience fever, it is common to treat such patients with antipyretics, such as aspirin, ibuprofen, naproxen, or acetaminophen. It is not uncommon for patients to show poor tolerance for systemic administration of certain NSAIDs, steroids and opiates. Moreover, several NSAIDs act as blood thinners and anticoagulants, which may increase the risk of postoperative hemorrhage.

There is therefore a need for an improved drug delivery device that is capable of delivering a drug having anti-inflammatory, analgesic, and/or antipyretic activity in the vicinity of a surgical implant over a prolonged period at a sustained concentration within the therapeutically effective concentration range for the drug.

### Summary of the Invention

Certain embodiments of the present invention provide a sustained release system comprising a polymer matrix and a prodrug, dispersed in the polymer, having a general formula of **A-L-B** in which: A represents a drug moiety having a therapeutically active form for producing a clinical response in a patient; L represents a covalent linker linking A and B to form a prodrug, said linker being cleaved under physiological conditions to generate said therapeutically active form of A; and B represents a moiety which, when linked to A, results in the prodrug having a lower solubility than the therapeutically active form of A. In certain embodiments, the linkage L is hydrolyzed in bodily fluid. In other embodiments, the linkage L is enzymatically cleaved. Examples of linkages which can be used include one or more hydrolyzable groups selected from the group consisting of an ester, an amide, a carbamate, a carbonate, a cyclic ketal, a thioester, a thioamide, a thiocarbamate, a thiocarbonate, a xanthate and a phosphate ester.

Other embodiments of the present invention provide a sustained release formulation comprising a polymer matrix and a prodrug, dispersed in the polymer, having a general formula of **A::B** in which A represents a drug moiety having a therapeutically active form for producing a clinical response in a patient; :: represents a ionic bond between A and B that dissociates under physiological conditions to generate said therapeutically active form of A; and B represents a moiety which, when ionically bonded to A, results in the prodrug having a lower solubility than the therapeutically active form of A.

In certain preferred embodiments, the solubility of therapeutically active form of A in water is greater than 1 mg/ml and the solubility of the prodrug in water is less than 1 mg/ml, and even more preferably less than 0.1 mg/ml, 0.01 mg/ml or even less than 0.001 mg/ml.

In certain preferred embodiments, the therapeutically active form of A is at least 10 times more soluble in water relative to said prodrug, and even more preferably at least 100, 1000 or even 10000 times more soluble in water relative to said prodrug.

In certain preferred embodiments, when disposed in biological fluid (such as serum, synovial fluid, cerebral spinal fluid, lymph, urine, etc), the sustained release formulation provides sustained release of the therapeutically active form of A for a period of at least 24 hours, and over that period of release, the concentration of the prodrug in fluid outside the polymer is less than 10% of the concentration of the therapeutically active form of A, and even more preferably less than 5%, 1% or even 0.1% of the concentration of the therapeutically active form of A.

In certain preferred embodiments, the therapeutically active form of A has a logP value at least 1 logP unit less than the logP value of the prodrug, and even more preferably at least 2, 3 or even 4 logP unit less than the logP value of the prodrug.

In certain preferred embodiments, the the prodrug, in its linked form, has an ED₅₀ for producing the clinical response at least 10 times greater than the ED₅₀ of the therapeutically active form of A, and even more preferably at least 100, 1000 or even 10000 times greater than the ED₅₀ of the therapeutically active form of A. That is, in many embodiments, the prodrug per se is inert with respect to inducing the clinical response.

In certain embodiments, B is a hydrophobic aliphatic moiety.

In some instances, B is drug moiety having a therapeutically active form generated upon cleavage of said linker L or dissociates of said ionic bond, and may be the same drug or a different drug than A.

In other embodiments, B, after cleavage from the prodrug, is a biologically inert moiety.

In many preferred embodiments, the duration of release from the polymer matrix of a therapeutically effective amount of the therapeutically active form of A is at least 24 hours, and even more preferably may be at least 72 hours, 100, 250, 500 or even 750 hours. In certain embodiments, the duration of release of the therapeutically active form of A from the polymer matrix is at least one week, more preferably two weeks, or even more preferably at least three weeks. In certain embodiments, the duration of release of the therapeutically active form of A from the polymer matrix is at least one month, more preferably two months, and even more preferably six months.

In certain embodiments, the pro-drug has an ED₅₀ at least 10 times greater than the ED₅₀ of the therapeutically active form of A. In preferred embodiments, the pro-drug has an ED₅₀ at least 100 times, or more preferably at least 1000 times, greater than the ED₅₀ of the therapeutically active form of A.

In some embodiments, the therapeutically active form of A is at least 10 times more soluble in water relative to said pro-drug. In preferred embodiments, the therapeutically active form of A is at least 100 times, or more preferably at least 1000 times, more soluble in water relative to said prodrug.

The A (and optionally B) moiety can be selected from amongst such drugs as immune response modifiers, anti-proliferatives, corticosteroids, angiostatic steroids, anti-parasitic drugs, anti-glaucoma drugs, antibiotics, anti-sense compounds, differentiation modulators, antiviral drugs, anticancer drugs, and non-steroidal anti-inflammatory drugs.

In certain embodiments, the polymer matrix is non-bioerodible, while in other embodiments it is bioerodible. Exemplary non-bioerodible polymer matrices can be formed from polyurethane, polysilicone, poly(ethylene-co-vinyl acetate), polyvinyl alcohol, and derivatives and copolymers thereof.

Exemplary bioerodible polymer matrices can be formed polyanhydride, polylactic acid, polyglycolic acid, polyorthoester, polyalkylcyanoacrylate, and derivatives and copolymers thereof.

In certain embodiments, the polymer matrix is chosen so as reduce interaction between the prodrug in the matrix and proteinaceous components in surrounding bathing fluid, e.g., by forming a matrix have physical (pore size, etc) and/or chemical (ionized groups, hydrophobicity, etc) characteristics which exclude proteins from the inner matrix, e.g., exclude proteins of greater than 100kD, and even more preferably exclude proteins greater in size than 50kD, 25kD, 10kD or even 5kD.

In certain embodiments, the polymer matrix is essentially non-release rate limiting with respect to the rate of release of the therapeutically active form of A from the matrix.

In other embodiments, the subject polymer matrices influence the rate of release. For instance, the matrices can be derived to have charge or hydrophobicity characteristics which favor sequestration of the prodrug over the released monomers (A and B). Likewise, the polymer matrix can influence the pH-dependency of the hydrolysis reaction, or create a microenvironment having a pH different than the bathing bodily fluid, such that hydrolysis and/or solubility of the prodrug is different within the matrix than in the surrounding fluids. In such a manner, the polymer can influence the rate of release, and the rate of hydrolysis of the prodrug, by differential electronic, hydrophobic or chemical interactions with the prodrug.

In certain embodiments, at least one of A or B is an antineoplastic agent. Exemplary antineoplastic agent include anthracyclines, vincaalkaloids, purine analogs, pyrimidine analogs, inhibitors of pyrimidine biosynthesis, and/or alkylating agents. Exemplary antineoplastic drugs include 5-fluorouracil (5FU), 5'-deoxy-5-fluorouridine 5-fluorouridine, 2'-deoxy-5-fluorouridine, fluorocytosine, 5-trifluoromethyl-2'-deoxyuridine, arabinoxyl cytosine, cyclocytidine, 5-aza-2'-deoxycytidine, arabinosyl 5-azacytosine, 6-azacytidine, N-phosphonoacetyl-L-aspartic acid, pyrazofurin, 6-azauridine, azaribine, 3-deazauridine, arabinosyl cytosine, cyclocytidine, 5-aza-2'-deoxycytidine, arabinosyl 5-azacytosine, 6-azacytidine, Cladribine, 6-mercaptopurine, pentostatin, 6-thioguanine, and fludarabin phosphate.

In certain preferred embodiments, the antineoplastic drug is a fluorinated pyrimidine, and even more preferably 5-fluorouracil, e.g., A is preferably 5-fluorouracil in certain embodiments.

In certain embodiments, at least one of A or B is an anti-inflammatory agent, such as, to illustrate, an a non-steroidal anti-inflammatory (diclofenac, fenoprofen, flurbiprofen, ibuprofen, ketoprofen, ketorolac, nahumstone, naproxen, piroxicam and the like) or an a glucocorticoid (such as aclometasone, beclomethasone, betamethasone, budesonide, clobetasol, clobetasone, cortisone, desonide, desoximetasone, diflorosane, flumethasone, flunisolide, fluocinolone acetonide, fluocinolone, fluocortolone, fluprednidene, flurandrenolide, fluticasone, hydrocortisone, methylprednisolone aceponate,mometasone furdate, prednisolone, prednisone and rofleponide).

In certain preferred embodiments, A is an antineoplastic fluorinated pyrimidine, such as 5-fluorouracil, and is an B is anti-inflammatory, such as fluocinolone acetonide, triamcinolone acetonide, diclofenac, or naproxen.

In some embodiments, the prodrug is selected from 5FU covalently bonded to fluocinolone acetonide (III), 5FU covalently bonded to naproxen (IV), and 5FU covalently bonded to diclofenac (V). Exemplary produgs include: 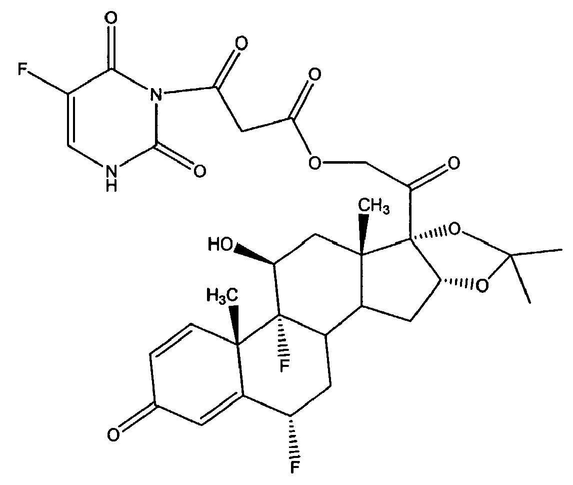 5FU-flucinolone acetonide (III), 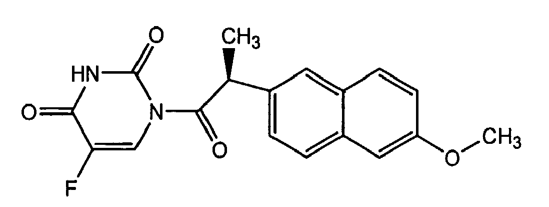 5FU-Naproxen (IV), and 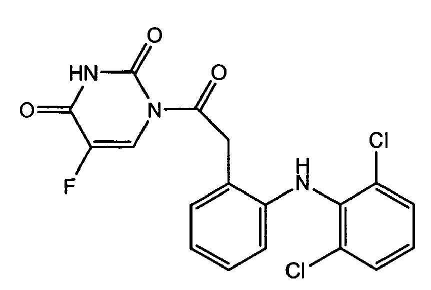 5FU-Diclofenac **(V).**

Another aspect of the invention relates to coated medical devices. For instance, in certain embodiments, the subject invention provides a medical device having a coating adhered to at least one surface, wherein the coating includes the subject polymer matrix and a low solubility prodrug. Such coatings can be applied to surgical implements such as screws, plates, washers, sutures, prosthesis anchors, tacks, staples, electrical leads, valves, membranes. The devices can be catheters, implantable vascular access ports, blood storage bags, blood tubing, central venous catheters, arterial catheters, vascular grafts, intraaortic balloon pumps, heart valves, cardiovascularsutures, artificial hearts, a pacemaker, ventricular assist pumps, extracorporeal devices, blood filters, hemodialysis units, hemoperfasion units, plasmapheresis units, and filters adapted for deployment in a blood vessel.

In a preferred embodiment, the subject coatings are applied to a vascular stent. In certain instances, particularly where the stent is an expandable stent, the coating is flexible to accommodate compressed and expanded states of the stent.

In certain embodiments, the weight of the coating attributable to the prodrug is in the range of about 0.05 mg to about 50 mg of prodrug per cm² of the surface coated with said polymer matrix, and even more preferably 5 to 25 mg/cm².

In certain embodiments, the coating has a thickness is in the range of 5 micrometers to 100 micrometers.

In certain embodiments, the prodrug is present in the coating in an amount between 5% and 70% by weight of the coating, and even more preferably 25 to 50% by weight.

Yet another aspect of the invention provides a method for treating an intraluminal tissue of a patient. In general, the method comprising the steps of:
(a) providing a stent having an interior surface and an exterior surface, said stent having a coating on at least a part of the interior surface, the exterior surface, or both; said coating comprising a low-solubility pharmaceutical prodrug dissolved or dispersed in a biologically-tolerated polymer;
(b) positioning the stent at an appropriate intraluminal tissue site; and
(c) deploying the stent.

Another aspect of the invention relates to a coating composition for use in delivering a medicament from the surface of a medical device positioned in vivo. The composition comprises a polymer matrix and low solubility prodrug as described above. The coating composition can be provided in liquid or suspension form for application to the surface of a medical device by spraying and/or dipping the device in the composition. In other embodiments, the coating composition is provided in powdered form and, upon addition of a solvent, can reconstitute a liquid or suspension form for application to the surface of a medical device by spraying and/or dipping the device in the composition.

Another aspect of the invention relates to an injectable composition for use in delivering a medicament to a patient. The composition includes a polymer matrix and low solubility prodrug as described above, and is provided in liquid or suspension form adapted for delivery by injection through a needle.

Additional advantages of the present invention will become readily apparent to those skilled in the art from the following detailed description, wherein only a preferred embodiment of the invention is shown and described by way of illustration of the best mode contemplated for carrying out the invention. As will be realized, the present invention is capable of other and different embodiments, and its several details are capable of modifications in various respects, all without departing from the scope of the present invention. Accordingly, the drawings and description are to be regarded as illustrative in nature, and not as restrictive.

### Brief Description Of The Drawings

FIG. 1 is a time-dependent graph of the release of a prodrug from a polymerprodrug dispersion according to the present invention.
FIG. 2 is a time-dependent graph of the release of a prodrug from a polymerprodrug dispersion according to the present invention.
FIG. 3 is a side plan view of a non-deployed stent according to the present invention.
FIG. 4 is a side plan view of a deployed stent according to the present invention.

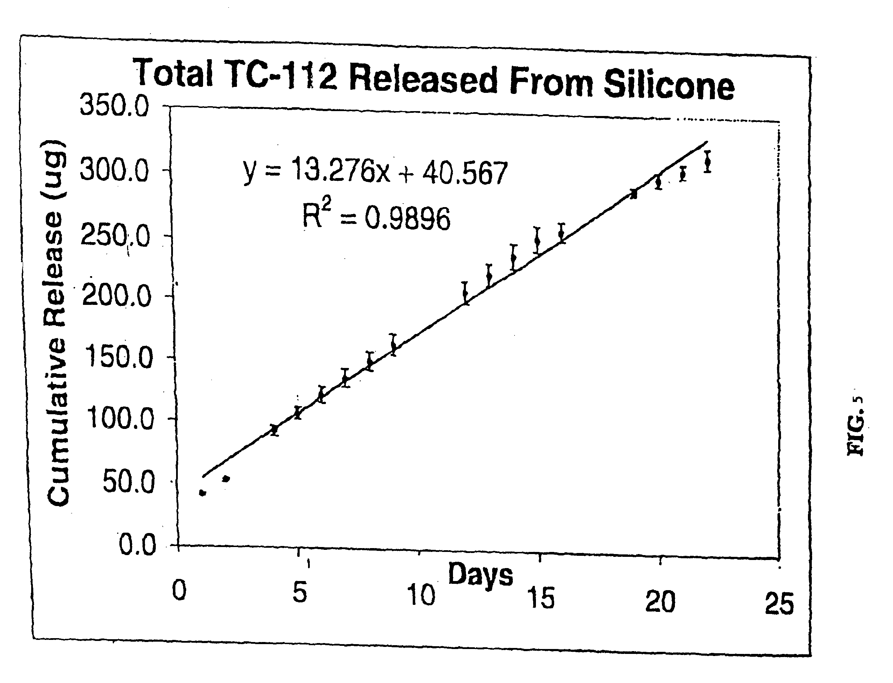

FIG. 5 is a release profile of TC-112 from PVA-coated glass slides into pH 7.4 buffer.

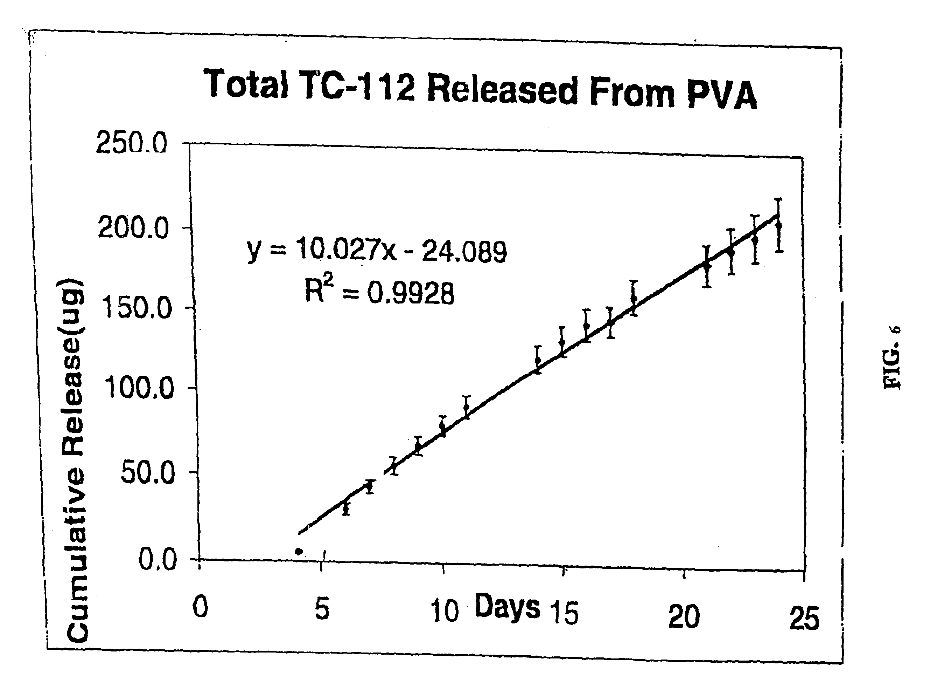

FIG. 6 is a release profile of TC-112 from silicone-coated glass plates into pH 7.4 buffer.
FIG. 7 is a release profile of 5-Fluroruracil (5FU) and triamcinolone acetonide (TA) from coated inserts.
FIG. 8 is a release profile of 5-Flurouracil (5FU) and triamcinolone acetonide (TA) from coated inserts.
FIG. 9 illustrate the release pattern in vitro for a High Dose coated stent.
FIG. 10 shows the comparative drug release profiles between explanted stents and non-implanted stents.
FIGS. 11A and 11B are graphs showing the effect of gamma irradiation and plasma treatment on drug release. Group B: with plasma treatment, with gamma irradiation. Group C: no plasma treatment, with gamma irradiation. Group D: with plasma treatment, no gamma irradiation. Group F: no plasma, no gamma irradiation

### Best Mode for Carrying Out the Invention

### Detailed Description Of The Invention

### I. Definitions

The term "active" as used herein means therapeutically or pharmacologically active.

The term "ED₅₀" means the dose of a drug that produces 50% of its maximum response or effect.

The term "IC₅₀" means the dose of a drug that inhibits a biological activity by 50%.

The term "LD₅₀" means the dose of a drug that is lethal in 50% of test subjects.

The term "therapeutic index" refers to the therapeutic index of a drug defined as LD₅₀/ED₅₀.

A "patient" or "subject" to be treated by the subject method can mean either a human or non-human animal.

"Physiological conditions" describe the conditions inside an organism, i.e., in vivo. Physiological conditions include the acidic and basic environments of body cavities and organs, enzymatic cleavage, metabolism, and other biological processes, and preferably refer to physiological conditions in a vertebrate, such as a mammal.

"LogP" refers to the logarithm of P (Partition Coefficient). P is a measure of how well a substance partitions between a lipid (oil) and water. P itself is a constant. It is defined as the ratio of concentration of compound in aqueous phase to the concentration of compound in an immiscible solvent, as the neutral molecule.

Partition Coefficient, P = [Organic] / [Aqueous] where [] = concentration LogP = log₁₀ (Partition Coefficient) = log₁₀P

In practice, the LogP value will vary according to the conditions under which it is measured and the choice of partitioning solvent. A LogP value of 1 means that the concentration of the compound is ten times greater in the organic phase than in the aqueous phase. The increase in a logP value of 1 indicates a ten fold increase in the concentration of the compound in the organic phase as compared to the aqueous phase. Thus, a compound with a logP value of 3 is 10 times more soluble in water than a compound with a logP value of 4 and a compound with a logP value of 3 is 100 times more soluble in water than a compound with a logP value of 5. In general, compounds having logP values between 7-10 are considered low solubility compounds.

### II. Exemplary Embodiments

The present invention provides a drug delivery system that can provide various release profiles, e.g., varying doses and/or varying lengths of time. The present invention thereby addresses the need for an insertable, injectable, inhalable, or implantable drug delivery system that provides controlled time-release kinetics of drug, particularly in the vicinity of a desired locus of drug activity, while avoiding complications associated with prior art devices.

The system of the present invention includes a polymer and a prodrug having a low solubility dispersed in the polymer. The polymer is permeable to the prodrug and is essentially non-release rate limiting with respect to the rate of release of the prodrug from the polymer, and provides sustained release of the drug.

Once administered, the system gives a continuous supply of the prodrug to the desired locus of activity without necessarily requiring additional invasive penetrations into these regions. Instead, the system remains in the body and serves as a continuous source of the prodrug to the affected area. The system according to the present invention permits prolonged release of drugs over a specific period of days, weeks, months (e.g., about 3 months to about 6 months) or years (e.g., about 1 year to about 20 years, such as from about 5 years to about 10 years) until the prodrug is used up.

The intraluminal medical device comprises the sustained release drug delivery coating. The inventive stent coating may be applied to the stent via a conventional coating process, such as impregnating coating, spray coating and dip coating.

In one embodiment, an intraluminal medical device comprises an elongate radially expandable tubular stent having an interior luminal surface and an opposite exterior surface extending along a longitudinal stent axis. The stent may include a permanent implantable stent, an implantable grafted stent, or a temporary stent, wherein the temporary stent is defined as a stent that is expandable inside a vessel and is thereafter retractable from the vessel. The stent configuration may comprise a coil stent, a memory coil stent, a Nitinol stent, a mesh stent, a scaffold stent, a sleeve stent, a permeable stent, a stent having a temperature sensor, a porous stent, and the like. The stent may be deployed according to conventional methodology, such as by an inflatable balloon catheter, by a self-deployment mechanism (after release from a catheter), or by other appropriate means. The elongate radially expandable tubular stent may be a grafted stent, wherein the grafted stent is a composite device having a stent inside or outside of a graft. The graft may be a vascular graft, such as an ePTFE graft, a biological graft, or a woven graft.

The drug combinations may be incorporated onto or affixed to the stent in a number of ways. In the exemplary embodiment, the drug combination is directly incorporated into a polymeric matrix and sprayed onto the outer surface of the stent. The drug combination elutes from the polymeric matrix over time and enters the surrounding tissue. The drug combination preferably remains on the stent for at least three days up to approximately six months, and more preferably between seven and thirty days.

The prodrugs are slowly dissolved in physiologic fluids, but are relatively quickly dissociated into at least one pharmaceutically active compound upon dissolution in physiologic fluids. In some embodiments the dissolution rate of the prodrug is in the range of about 0.001 µg/day to about 10 µg/day. In certain embodiments, the prodrugs have dissolution rates in the range of about 0.01 to about 1 µg/day. In particular embodiments, the prodrugs have dissolution rates of about 0.1 µg/day.

The low-solubility pharmaceutical prodrug is incorporated into a biocompatable (i.e., biologically tolerated) polymer vehicle. In some embodiments according to the present invention, the low-solubility pharmaceutical prodrug is present as a plurality of granules dispersed within the polymer vehicle. In such cases, it is preferred that the low-solubility pharmaceutical prodrug be relatively insoluble in the polymer vehicle, however the low-solubility pharmaceutical prodrug may possess a finite solubility coefficient with respect to the polymer vehicle and still be within the scope of the present invention. In either case, the polymer vehicle solubility of the low-solubility pharmaceutical prodrug should be such that the prodrug will disperse throughout the polymer vehicle, while remaining in substantially granular form.

In some embodiments according to the present invention, the low-solubility pharmaceutical prodrug is dissolved within the polymer vehicle. In such cases, it is preferred that the polymer vehicle be a relatively non-polar or hydrophobic polymer which acts as a good solvent for the relatively hydrophobic low-solubility pharmaceutical prodrug. In such cases, the solubility of the low-solubility pharmaceutical prodrug in the polymer vehicle should be such that the prodrug will dissolve thoroughly in the polymer vehicle, being distributed homogeneously throughout the polymer vehicle.

The polymer according to the present invention comprises any biologically tolerated polymer that is permeable to the prodrug and while having a permeability such that it is not the principal rate determining factor in the rate of release of the prodrug from the polymer.

In some embodiments according to the present invention, the polymer is non-bioerodible. Examples of non-bioerodible polymers useful in the present invention include poly(ethylene-co-vinyl acetate) (EVA), polyvinylalcohol and polyurethanes, such as polycarbonate-based polyurethanes. In other embodiments of the present invention, the polymer is bioerodible. Examples of bioerodible polymers useful in the present invention include polyanhydride, polylactic acid, polyglycolic acid, polyorthoester, polyalkylcyanoacrylate or derivatives and copolymers thereof. The skilled artisan will recognize that the choice of bioerodibility or non-bioerodibility of the polymer depends upon the final physical form of the system, as described in greater detail below. Other exemplary polymers include polysilicone and polymers derived from hyaluronic acid. The skilled artisan will understand that the polymer according to the present invention is prepared under conditions suitable to impart permeability such that it is not the principal rate determining factor in the release of the low solubility prodrug from the polymer.

Moreover, suitable polymers include naturally occurring (collagen, hyaluronic acid, etc.) or synthetic materials that are biologically compatible with bodily fluids and mammalian tissues, and essentially insoluble in bodily fluids with which the polymer will come in contact. In addition, the suitable polymers essentially prevent interaction between the low solubility prodrug dispersed/suspended in the polymer and proteinaceous components in the bodily fluid. The use of rapidly dissolving polymers or polymers highly soluble in bodily fluid or which permit interaction between the low solubility prodrug and proteinaceous components are to be avoided in certain instances since dissolution of the polymer or interaction with proteinaceous components would affect the constancy of drug release.

Other suitable polymers include polypropylene, polyester, polyethylene vinyl acetate (PVA or EVA), polyethylene oxide (PEO), polypropylene oxide, polycarboxylic acids, polyalkylacrylates, cellulose ethers, silicone, poly(dl-lactide-co glycolide), various Eudragrits (for example, NE30D, RS PO and RL PO), polyalkylalkyacrylate copolymers, polyester-polyurethane block copolymers, polyetherpolyurethane block copolymers, polydioxanone, poly-(β-hydroxybutyrate), polylactic acid (PLA), polycaprolactone, polyglycolic acid, and PEO-PLA copolymers.

The coating of the present invention may be formed by mixing one or more suitable monomers and a suitable low-solubility pharmaceutical prodrug, then polymerizing the monomer to form the polymer system. In this way, the prodrug is dissolved or dispersed in the polymer. In other embodiments, the prodrug is mixed into a liquid polymer or polymer dispersion and then the polymer is further processed to form the inventive coating. Suitable further processing may include crosslinking with suitable crosslinking prodrugs, further polymerization of the liquid polymer or polymer dispersion, copolymerization with a suitable monomer, block copolymerization with suitable polymer blocks, etc. The further processing traps the drug in the polymer so that the drug is suspended or dispersed in the polymer vehicle.

Any number of non-erodible polymers may be utilized in conjunction with the drug combination. Film-forming polymers that can be used for coatings in this application can be absorbable or non-absorbable and must be biocompatible to minimize irritation to the vessel wall. The polymer may be either biostable or bioabsorbable depending on the desired rate of release or the desired degree of polymer stability, but a bioabsorbable polymer may be preferred since, unlike biostable polymer, it will not be present long after implantation to cause any adverse, chronic local response. Furthermore, bioabsorbable polymers do not present the risk that over extended periods of time there could be an adhesion loss between the stent and coating caused by the stresses of the biological environment that could dislodge the coating and introduce further problems even after the stent is encapsulated in tissue.

Suitable film-forming bioabsorbable polymers that could be used include polymers selected from the group consisting of aliphatic polyesters, poly(amino acids), copoly(ether-esters), polyalkylenes oxalates, polyamides, poly(iminocarbonates), polyorthoesters, polyoxaesters, polyamidoesters, polyoxaesters containing amido groups, poly(anhydrides), polyphosphazenes, biomolecules and blends thereof. For the purpose of this invention aliphatic polyesters include homopolymers and copolymers of lactide (which includes lactic acid d-,1- and meso lactide), ε-caprolactone, glycolide (including glycolic acid), hydroxybutyrate, hydroxyvalerate, para-dioxanone, trimethylene carbonate (and its alkyl derivatives), 1,4-dioxepan-2-one, 1,5-dioxepan-2-one, 6,6-dimethyl-1,4-dioxan-2-one and polymer blends thereof. Poly(iminocarbonate) for the purpose of this invention include as described by Kemnitzer and Kohn, in the Handbook of Biodegradable Polymers, edited by Domb, Kost and Wisemen, Hardwood Academic Press, 1997, pages 251-272. Copoly(ether-esters) for the purpose of this invention include those copolyester-ethers described in Journal of Biomaterials Research, Vol. 22, pages 993-1009, 1988 by Cohn and Younes and Cohn, Polymer Preprints (ACS Division of Polymer Chemistry) Vol. 30(1), page 498, 1989 (e.g. PEO/PLA). Polyalkylene oxalates for the purpose of this invention include U.S. Pat. Nos. 4,208,511; 4,141,087; 4,130,639; 4,140,678; 4,105,034; and 4,205,399 (incorporated by reference herein). Polyphosphazenes, co-, ter- and higher order mixed monomer based polymers made from L-lactide, D,L-lactide, lactic acid, glycolide, glycolic acid, para-dioxanone, trimethylene carbonate and ε-caprolactone such as are described by Allcock in The Encyclopedia of Polymer Science, Vol. 13, pages 31-41, Wiley Intersciences, John Wiley & Sons, 1988 and by Vandorpe, Schacht, Dejardin and Lemmouchi in the Handbook of Biodegradable Polymers, edited by Domb, Kost and Wisemen, Hardwood Academic Press, 1997, pages 161-182 (which are hereby incorporated by reference herein). Polyanhydrides from diacids of the form HOOC-C₆H₄-O-(CH₂)ₘ-O-C₆H₄-COOH where m is an integer in the range of from 2 to 8 and copolymers thereof with aliphatic alpha-omega diacids of up to 12 carbons. Polyoxaesters polyoxaamides and polyoxaesters containing amines and/or amido groups are described in one or more of the following U.S. Pat. Nos. 5,464,929; 5,595,751; 5,597,579; 5,607,687; 5,618,552; 5,620,698; 5,645,850; 5,648,088; 5,698,213 and 5,700,583; (which are incorporated herein by reference). Polyorthoesters such as those described by Heller in Handbook of Biodegradable Polymers, edited by Domb, Kost and Wisemen, Hardwood Academic Press, 1997, pages 99-118 (hereby incorporated herein by reference). Film-forming polymeric biomolecules for the purpose of this invention include naturally occurring materials that may be enzymatically degraded in the human body or are hydrolytically unstable in the human body such as fibrin, fibrinogen, collagen, elastin, and absorbable biocompatable polysaccharides such as chitosan, starch, fatty acids (and esters thereof), glucoso-glycans and hyaluronic acid.

Suitable film-forming biostable polymers with relatively low chronic tissue response, such as polyurethanes, silicones, poly(meth)acrylates, polyesters, polyalkyl oxides (polyethylene oxide), polyvinyl alcohols, polyethylene glycols and polyvinyl pyrrolidone, as well as, hydrogels such as those formed from crosslinked polyvinyl pyrrolidinone and polyesters could also be used. Other polymers could also be used if they can be dissolved, cured or polymerized on the stent. These include polyolefins, polyisobutylene and ethylene-alphaolefin copolymers; acrylic polymers (including methacrylate) and copolymers, vinyl halide polymers and copolymers, such as polyvinyl chloride; polyvinyl ethers, such as polyvinyl methyl ether; polyvinylidene halides such as polyvinylidene fluoride and polyvinylidene chloride; polyacrylonitrile, polyvinyl ketones; polyvinyl aromatics such as polystyrene; polyvinyl esters such as polyvinyl acetate; copolymers of vinyl monomers with each other and olefins, such as etheylene-methyl methacrylate copolymers, acrylonitrilestyrene copolymers, ABS resins and ethylene-vinyl acetate copolymers; polyamides,such as Nylon 66 and polycaprolactam; alkyd resins; polycarbonates; polyoxymethylenes; polyimides; polyethers; epoxy resins, polyurethanes; rayon; rayon-triacetate, cellulose, cellulose acetate, cellulose acetate butyrate; cellophane; cellulose nitrate; cellulose propionate; cellulose ethers (i.e. carboxymethyl cellulose and hydoxyalkyl celluloses); and combinations thereof. Polyamides for the purpose of this application would also include polyamides of the form -NH-(CH₂)ₙ-CO- and NH-(CH₂)ₓ-NH-CO-(CH₂)_{y}-CO, wherein n is preferably an integer in from 6 to 13; x is an integer in the range of form 6 to 12; and y is an integer in the range of from 4 to 16. The list provided above is illustrative but not limiting.

The polymers used for coatings can be film-forming polymers that have molecular weight high enough as to not be waxy or tacky. The polymers also should adhere to the stent and should not be so readily deformable after deposition on the stent as to be able to be displaced by hemodynamic stresses. The polymers molecular weight be high enough to provide sufficient toughness so that the polymers will not to be rubbed off during handling or deployment of the stent and must not crack during expansion of the stent. In certain embodiments, the polymer has a melting temperature above 40°C, preferably above about 45°C, more preferably above 50°C and most preferably above 55°C.

Coating may be formulated by mixing one or more of the therapeutic prodrugs with the coating polymers in a coating mixture. The therapeutic prodrug may be present as a liquid, a finely divided solid, or any other appropriate physical form. Optionally, the mixture may include one or more additives, e.g., nontoxic auxiliary substances such as diluents, carriers, excipients, stabilizers or the like. Other suitable additives may be formulated with the polymer and pharmaceutically active prodrug or compound. For example, hydrophilic polymers selected from the previously described lists ofbiocompatible film forming polymers may be added to a biocompatible hydrophobic coating to modify the release profile (or a hydrophobic polymer may be added to a hydrophilic coating to modify the release profile). One example would be adding a hydrophilic polymer selected from the group consisting of polyethylene oxide, polyvinyl pyrrolidone, polyethylene glycol, carboxylmethyl cellulose, hydroxymethyl cellulose and combination thereof to an aliphatic polyester coating to modify the release profile. Appropriate relative amounts can be determined by monitoring the in vitro and/or in vivo release profiles for the therapeutic prodrugs.

The thickness of the coating can determine the rate at which the active drug(s) or prodrug elutes from the matrix. Essentially, the active drug(s) or prodrug elutes from the matrix by diffusion through the polymer matrix. Polymers are permeable, thereby allowing solids, liquids and gases to escape therefrom. The total thickness of the polymeric matrix is in the range from about one micron to about twenty microns or greater. It is important to note that primer layers and metal surface treatments may be utilized before the polymeric matrix is affixed to the medical device. For example, acid cleaning, alkaline (base) cleaning, salinization and parylene deposition may be used as part of the overall process described.

In certain embodiments, multiple coatings can be used. For instance, the various coatings can differ in the concentration of prodrug, the identity of the prodrugs (active ingredients, linkers, etc), the characteristics of the polymer matrix (composition, porosity, etc) and/or the presence of other drugs or release modifiers.

To further illustrate, a poly(ethylene-co-vinylacetate), polybutylmethacrylate and drug combination solution may be incorporated into or onto the stent in a number of ways. For example, the solution may be sprayed onto the stent or the stent may be dipped into the solution. Other methods include spin coating and RF plasma polymerization. In one exemplary embodiment, the solution is sprayed onto the stent and then allowed to dry. In another exemplary embodiment, the solution may be electrically charged to one polarity and the stent electrically changed to the opposite polarity. In this manner, the solution and stent will be attracted to one another. In using this type of spraying process, waste may be reduced and more precise control over the thickness of the coat may be achieved.

In another exemplary embodiment, the drug combination or other therapeutic prodrug may be incorporated into a film-forming polyfluoro copolymer comprising an amount of a first moiety selected from the group consisting of polymerized vinylidenefluoride and polymerized tetrafluoroethylene, and an amount of a second moiety other than the first moiety and which is copolymerized with the first moiety, thereby producing the polyfluoro copolymer, the second moiety being capable of providing toughness or elastomeric properties to the polyfluoro copolymer, wherein the relative amounts of the first moiety and the second moiety are effective to provide the coating and film produced therefrom with properties effective for use in treating implantable medical devices.

In one embodiment according to the present invention, the exterior surface of the expandable tubular stent of the intraluminal medical device of the present invention comprises a coating according to the present invention. The exterior surface of a stent having a coating is the tissue-contacting surface and is biocompatible. The "sustained release drug delivery system coated surface" is synonymous with "coated surface", which surface is coated, covered or impregnated with sustained release drug delivery system according to the present invention.

In an alternate embodiment, the interior luminal surface or entire surface (i.e. both interior and exterior surfaces) of the elongate radially expandable tubular stent of the intraluminal medical device of the present invention has the coated surface. The interior luminal surface having the inventive sustained release drug delivery system coating is also the fluid contacting surface, and is biocompatible and blood compatible.

U.S. Pat. No. 5,773,019, U.S. Pat. No. 6,001,386, and U.S. Pat. No. 6,051,576 disclose implantable controlled-release devices and drugs and are incorporated in their entireties herein by reference. The inventive process for making a surface coated stent includes deposition onto the stent of a coating by, for example, dip coating or spray coating. In the case of coating one side of the stent, only the surface to be coated is exposed to the dip or spray. The treated surface may be all or part of an interior luminal surface, an exterior surface, or both interior and exterior surfaces of the intraluminal medical device. The stent may be made of a porous material to enhance deposition or coating into a plurality of micropores on or in the applicable stent surface, wherein the microporous size is preferably about 100 microns or less.

Problems associated with treating restinosis and neointimal hyperplasia can be addressed by the choice of pharmaceutical prodrug used to coat the medical device. In certain preferred embodiments of the present invention, the chosen pharmaceutical prodrug is a moiety of low-solubility and comprises at least two pharmaceutically active compounds. The pharmaceutically active compounds can be the same or different chemical species, and can be formed, as desired, in equi-molar or non-equi-molar concentrations to provide optimal treatment based on the relative activities and other pharmaco-kinetic properties of the compounds. The drug combination, particularly where co-drug formulations are used, may itself be advantageously relatively insoluble in physiologic fluids, such as blood and blood plasma, and has the property of regenerating any or all of the pharmaceutically active compounds when dissolved in physiologic fluids. In other words, to the extent that the low-solubility prodrug dissolves in physiologic fluids, it is quickly and efficiently converted into the constituent pharmaceutically active compounds upon dissolution. The low-solubility of the pharmaceutical prodrug thus insures persistence of the prodrug in the vicinity of an intraluminal lesion. The quick conversion of the low-solubility pharmaceutical prodrug into the constituent pharmaceutically active compounds insures a steady, controlled, dose of the pharmaceutically active compounds near the site of the lesion to be treated.

Examples of a suitable first pharmaceutically active compound include immune response modifiers such as cyclosporin A and FK 506, corticosteroids such as dexamethasone, fluocinolone acetonide and triamcinolone acetonide, angiostatic steroids such as trihydroxy steroids, antibiotics including ciprofloxacin, differentiation modulators such as retinoids (e.g., trans-retinoic acid, cis-retinoic acid and analogues), anticancer/anti-proliferative prodrugs such as 5-fluorouracil (5FU) and BCNU, and non-steroidal anti-inflammatory prodrugs such as naproxen, diclofenac, indomethacin and flurbiprofen.

In some embodiments according to the present invention, the preferred first pharmaceutically active compound is 5FU.

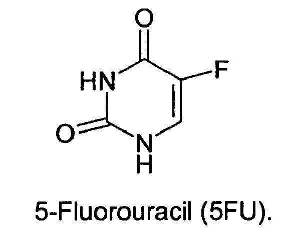

Examples of a suitable second pharmaceutically active compound include immune response modifiers such as cyclosporin A and FK 506, corticosteroids such as dexamethasone, fluocinolone acetonide and triamcinolone acetonide, angiostatic steroids such as trihydroxy steroids, antibiotics including ciprofloxacin, differentiation modulators such as retinoids (e.g., trans-retinoic acid, cis-retinoic acid and analogues), anticancer/anti-proliferative prodrugs such as 5-fluorouracil (5FU) and BCNU, and non-steroidal anti-inflammatory prodrugs such as naproxen, diclofenac, indomethacin and flurbiprofen.

In some embodiments according to the present invention, the second pharmaceutically active compound is selected from fluocinolone acetonide, triamcinolone acetonide, diclofenac, and naproxen.

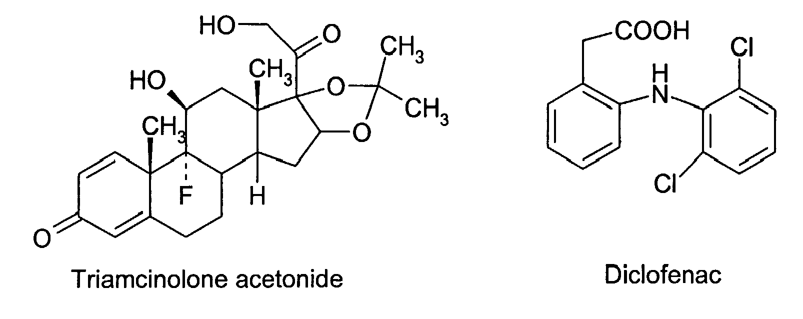

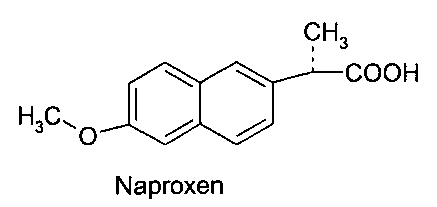

The low-solubility pharmaceutically active prodrug according to the present invention may comprise further residues of pharmaceutically active compounds. Such further pharmaceutically active compounds include immune response modifiers such as cyclosporin A and FK 506, corticosteroids such as dexamethasone, fluocinolone acetonide and triamcinolone acetonide, angiostatic steroids such as trihydroxy steroids, antibiotics including ciprofloxacin, differentiation modulators such as retinoids (e.g., trans-retinoic acid, cis-retinoic acid and analogues), anticancer/anti-proliferative prodrugs such as 5-fluorouracil (5FU) and BCNU, and non-steroidal anti-inflammatory prodrugs such as naproxen, diclofenac, indomethacin and flurbiprofen.

In certain embodiments, the low-solubility pharmaceutical prodrug comprises a moiety of at least two pharmaceutically active compounds that can be covalently bonded, connected through a linker, ionically combined, or combined as a mixture.

In some embodiments according to the present invention, the first and second pharmaceutically active compounds are covalently bonded directly to one another. Where the first and second pharmaceutically active compounds are directly bonded to one another by a covalent bond, the bond may be formed by forming a suitable covalent linkage through an active group on each active compound. For instance, an acid group on the first pharmaceutically active compound may be condensed with an amine, an acid or an alcohol on the second pharmaceutically active compound to form the corresponding amide, anhydride or ester, respectively.

In addition to carboxylic acid groups, amine groups, and hydroxyl groups, other suitable active groups for forming linkages between pharmaceutically active moieties include sulfonyl groups, sulfhydryl groups, and the haloic acid and acid anhydride derivatives of carboxylic acids.

In other embodiments, the pharmaceutically active compounds may be covalently linked to one another through an intermediate linker. The linker advantageously possesses two active groups, one of which is complementary to an active group on the first pharmaceutically active compound, and the other of which is complementary to an active group on the second pharmaceutically active compound. For example, where the first and second pharmaceutically active compounds both possess free hydroxyl groups, the linker may suitably be a diacid, which will react with both compounds to form a diether linkage between the two residues. In addition to carboxylic acid groups, amine groups, and hydroxyl groups, other suitable active groups for forming linkages between pharmaceutically active moieties include sulfonyl groups, sulfhydryl groups, and the haloic acid and acid anhydride derivatives of carboxylic acids.

Suitable linkers are set forth in Table 1 below.

**Table 1**

| First Pharmaceutically Active Compound Active Group | Second Pharmaceutically Active Compound Active Group | Suitable Linker |
|---|---|---|
| Amine | Amine | Diacid |
| Amine | Hydroxy | Diacid |
| Hydroxy | Amine | Diacid |
| Hydroxy | Hydroxy | Diacid |
| Acid | Acid | Diamine |
| Acid | Hydroxy | Amino acid, hydroxyalk acid, sulfhydrylalkyl acid |
| Acid | Amine | Amino acid, hydroxyalk acid, sulfhydrylalkyl acid |

Suitable diacid linkers include oxalic, malonic, succinic, glutaric, adipic, pimelic, suberic, azelaic, sebacic, maleic, fumaric, tartaric, phthalic, isophthalic, and terephthalic acids. While diacids are named, the skilled artisan will recognize that in certain circumstances the corresponding acid halides or acid anhydrides (either unilateral or bilateral) are preferred as linker reprodrugs. A preferred anhydride is succinic anhydride. Another preferred anhydride is maleic anhydride. Other anhydrides and/or acid halides may be employed by the skilled artisan to good effect.

Suitable amino acids include γ-butyric acid, 2-aminoacetic acid, 3-aminopropanoic acid, 4-aminobutanoic acid, 5-aminopentanoic acid, 6-aminohexanoic acid, alanine, arginine, asparagine, aspartic acid, cysteine, glutamic acid, glutamine, glycine, histidine, isoleucine, leucine, lysine, methionine, phenylalanine, proline, serine, threonine, tryptophan, tyrosine, and valine. Again, the acid group of the suitable amino acids may be converted to the anhydride or acid halide form prior to their use as linker groups.

Suitable diamines include 1, 2-diaminoethane, 1,3-diaminopropane, 1,4-diaminobutane, 1,5-diaminopentane, 1,6-diaminohexane.

Suitable aminoalcohols include 2-hydroxy-l-aminoethane, 3-hydroxy-1-aminoethane, 4-hydroxy-l-aminobutane, 5-hydroxy-1-aminopentane, 6-hydroxy-1-aminohexane.

Suitable hydroxyalkyl acids include 2-hydroxyacetic acid, 3-hydroxypropanoic acid, 4-hydroxybutanoic acid, 5-hydroxypentanoic acid, 5-hydroxyhexanoic acid.

The person having skill in the art will recognize that by selecting first and second pharmaceutical moieties (and optionally third, etc. pharmaceutical moieties) having suitable active groups, and by matching them to suitable linkers, a broad palette of inventive compounds may be prepared within the scope of the present invention.

Exemplary preferred low-solubility pharmaceutically active prodrugs include 5FU covalently bonded to fluocinolone acetonide, 5FU covalently bonded to diclofenac, and 5FU covalently bonded to naproxen. Illustrative examples include the following:

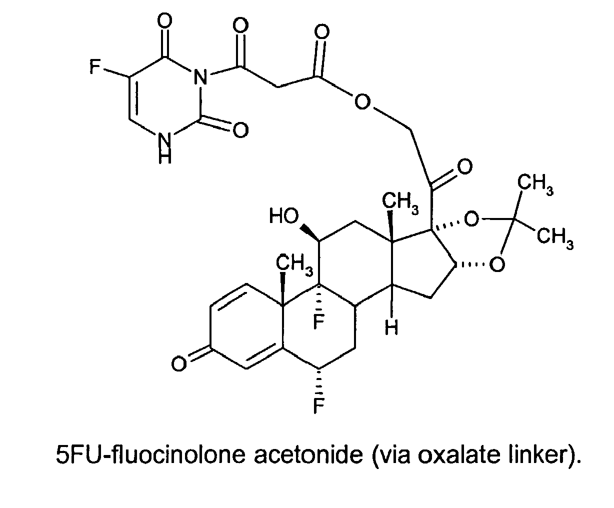

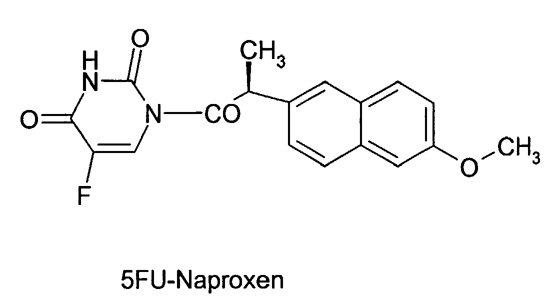

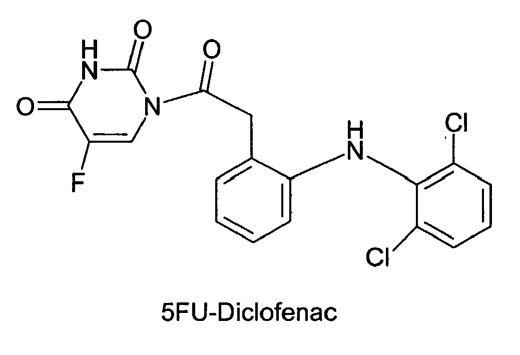

Other exemplary codrugs include the following:

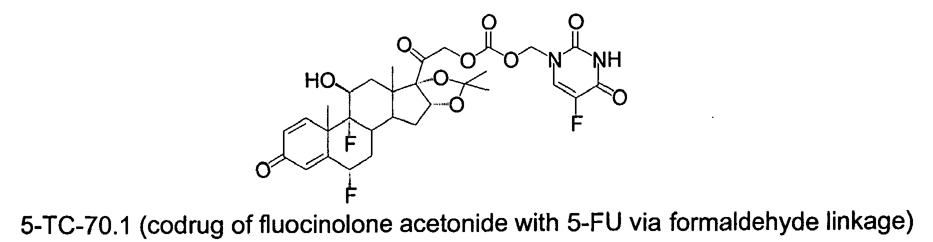

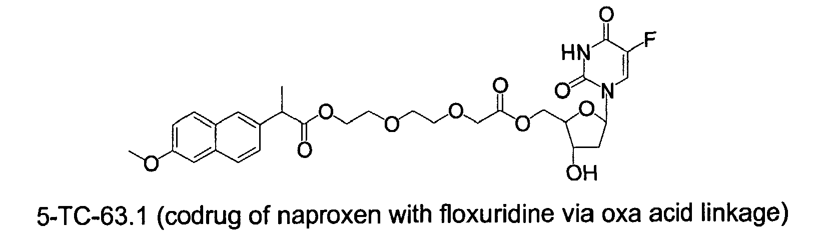

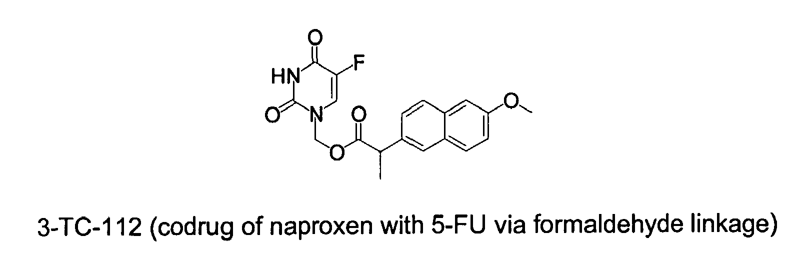

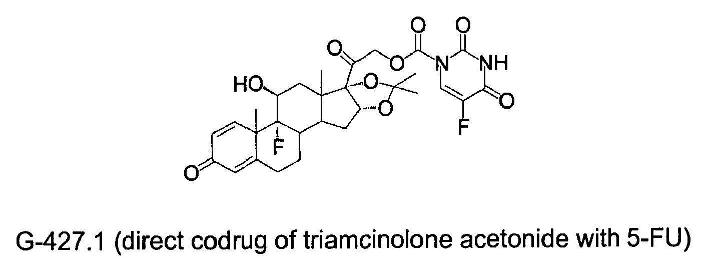

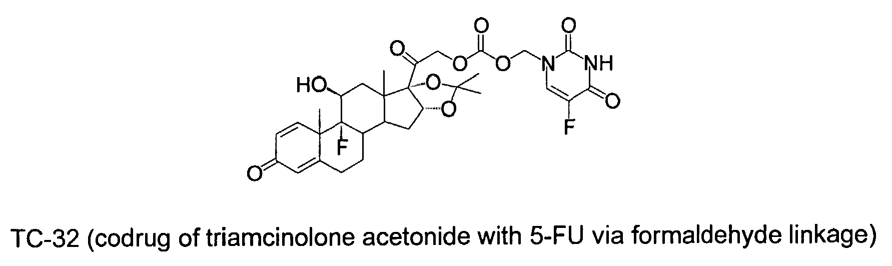

Some exemplary co-drugs which join the first and second pharmaceutically active compounds with different linkages include:

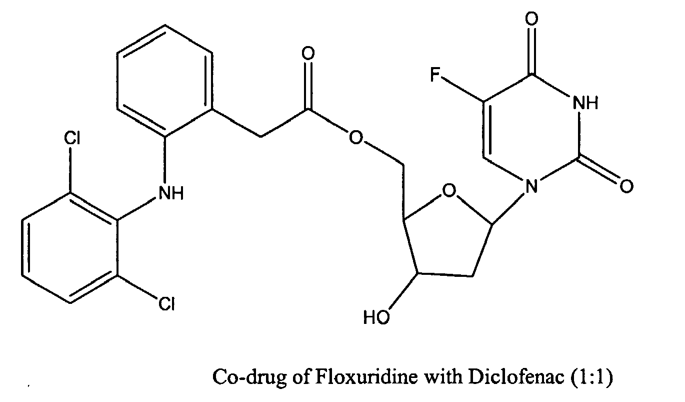

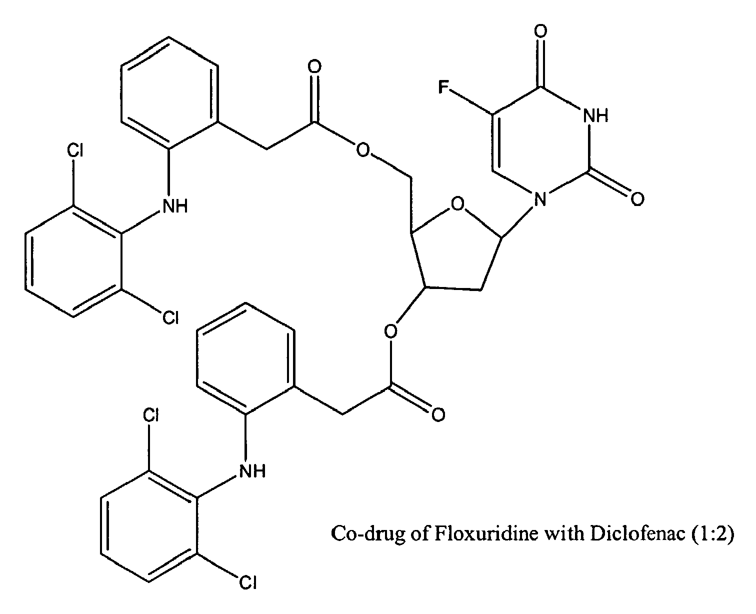

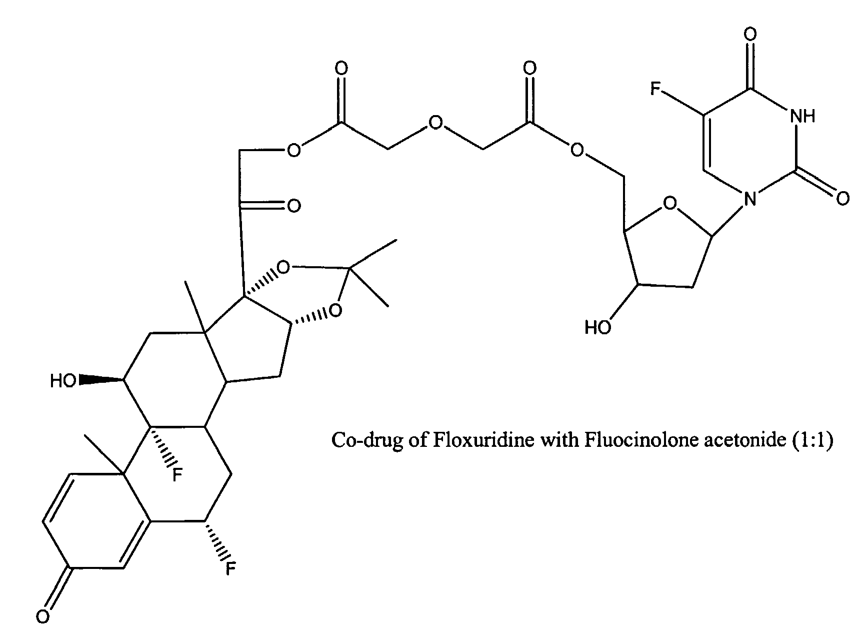

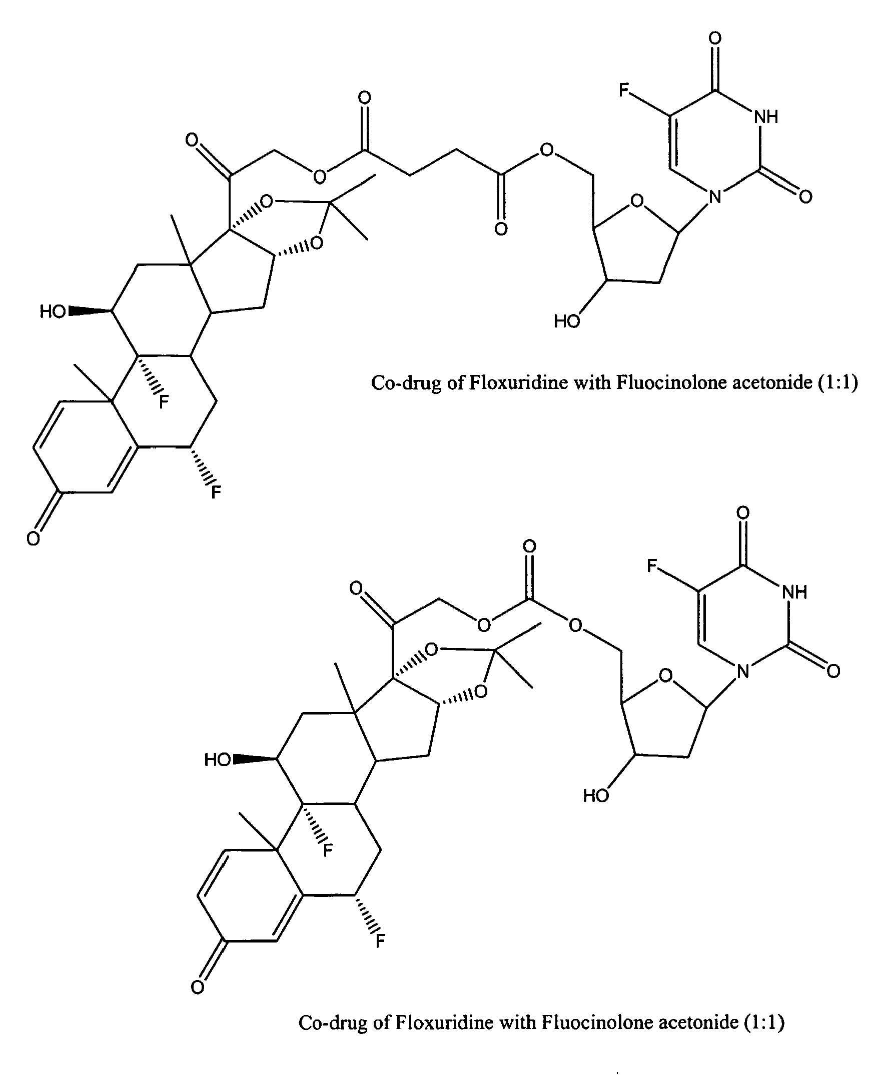

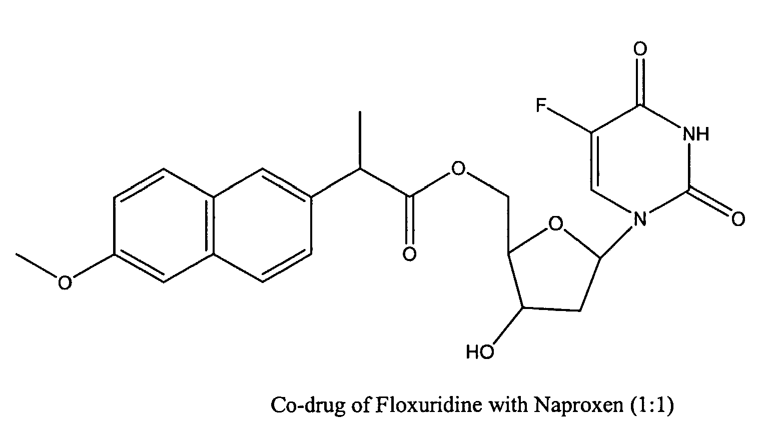

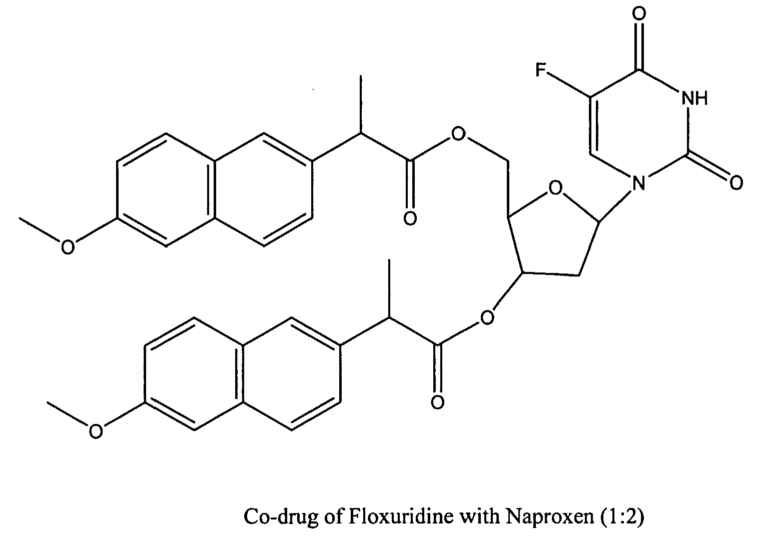

In other embodiments, the first and second pharmaceutically active compounds may be combined to form a salt. For instance, the first pharmaceutically active compound may be an acid, and the second pharmaceutically active compound may be a base, such as an amine. As a specific example, the first pharmaceutically active compound may be diclofenac or naproxen (acids), and the second pharmaceutically active compound may be ciprofloxacin (a base). The combination of diclofenac and ciprofloxacin would for instance form the salt:

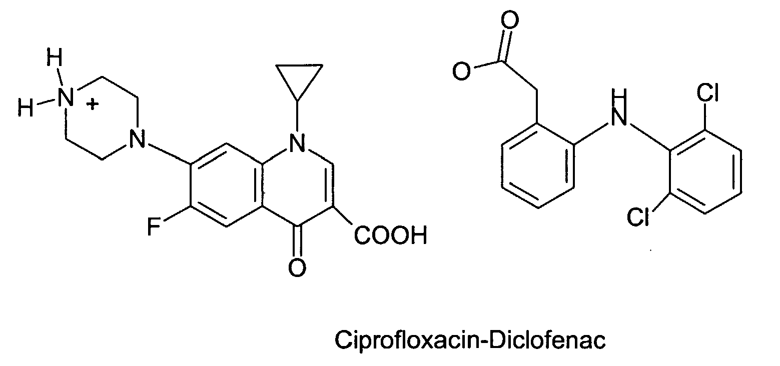

For the system of present invention to deliver a prodrug in desired fashion, e.g., constant or substantially linear in some embodiments, he solubility of the drug and the permeability of the polymer must be balanced so that the permeability of the polymer is not the principal rate determining factor in the delivery of the drug. As a result, the rate of release of the prodrug is essentially the rate at which the prodrug is solubilized in the surrounding aqueous medium. This rate of release is nearly approximately linear with respect to time (so-called zero-order kinetics.).

The system of the present invention may be formed by mixing one or more suitable monomers and a suitable low-solubility pharmaceutical prodrug, then polymerizing the monomer to form the polymer system. In this way, the prodrug is dissolved or dispersed in the polymer. In other embodiments, the prodrug is mixed into a liquid polymer or polymer dispersion and then the polymer is further processed to form the inventive system. Suitable further processing includes crosslinking with suitable crosslinking prodrugs, further polymerization of the liquid polymer or polymer dispersion, copolymerization with a suitable monomer, block copolymerization with suitable polymer blocks, etc. The further processing traps the drug in the polymer so that the drug is suspended or dispersed in the polymer vehicle.

In some embodiments according to the present invention, monomers for forming a polymer are combined with an inventive low-solubility compound and are mixed to make a homogeneous dispersion of the inventive compound in the monomer solution. The dispersion is then applied to a stent according to a conventional coating process, after which the crosslinking process is initiated by a conventional initiator, such as UV light. In other embodiments according to the present invention, a polymer composition is combined with an inventive low-solubility compound to form a dispersion. The dispersion is then applied to a stent and the polymer is cross-linked to form a solid coating. In other embodiments according to the present invention, a polymer and an inventive low-solubility compound are combined with a suitable solvent to form a dispersion, which is then applied to a stent in a conventional fashion. The solvent is then removed by a conventional process, such as heat evaporation, with the result that the polymer and inventive low-solubility drug (together forming a sustained-release drug delivery system) remain on the stent as a coating. An analogous process may be used where the inventive low-solubility pharmaceutical compound is dissolved in the polymer composition.

In some embodiments according to the invention, the system comprises a polymer that is relatively rigid. In other embodiments, the system comprises a polymer that is soft and malleable. In still other embodiments, the system includes a polymer that has an adhesive character. Hardness, elasticity, adhesive, and other characteristics of the polymer are widely variable, depending upon the particular final physical form of the system, as discussed in more detail below.

Embodiments of the system according to the present invention take many different forms. In some embodiments, the system consists of the low solubility prodrug, i.e., the prodrug suspended or dispersed in the polymer. In certain other embodiments, the system consists of a prodrug and a semi-solid or gel polymer, which is adapted to be injected via a syringe into a body. In other embodiments according to the present invention, the system consists of a prodrug and a soft-flexible polymer, which is adapted to be inserted or implanted into a body by a suitable surgical method. In still further embodiments according to the present invention, the system consists of a hard, solid polymer, which is adapted to be inserted or implanted into a body by a suitable surgical method. In additional embodiments of the present invention, the system comprises a polymer having the low solubility prodrug suspended or dispersed therein which is suitable for inhalation. In further embodiments, the system comprises a polymer having the prodrug suspended or dispersed therein, wherein the prodrug and polymer mixture forms a coating on a surgical implement, such as a screw, stent, pacemaker, etc. In particular embodiments according to the present invention, the device consists of a hard, solid polymer, which is shaped in the form of a surgical implement such as a surgical screw, plate, stent, etc., or some part thereof. In other embodiments according to the present invention, the system includes a polymer that is in the form of a suture having the drug dispersed or suspended therein.

In some embodiments according to the present invention, provided is a medical device comprising a substrate having a surface, such as an exterior surface, and a coating on the exterior surface. The coating comprises a polymer and a prodrug having a low solubility dispersed in the polymer, wherein the polymer is permeable to the prodrug and is essentially non-release rate limiting with respect to the rate of release of the prodrug from the polymer. In certain embodiments according to the present invention, the device comprises a prodrug suspended or dispersed in a suitable polymer, wherein the prodrug and polymer are coated onto an entire substrate, e.g., a surgical implement. Such coating may be accomplished by spray coating or dip coating.

In other embodiments according to the present invention, the device comprises a prodrug and polymer suspension or dispersion, wherein the polymer is rigid, and forms a constituent part of a device to be inserted or implanted into a body. For instance, in particular embodiments according to the present invention, the device is a surgical screw, stent, pacemaker, etc. coated with the prodrug suspended or dispersed in the polymer. In other particular embodiments according to the present invention, the polymer in which the prodrug is suspended forms a tip or a head, or part thereof, of a surgical screw. In other embodiments according to the present invention, the polymer in which prodrug is suspended or dispersed is coated onto a surgical implement such as surgical tubing (such as colostomy, peritoneal lavage, catheter, and intravenous tubing). In still further embodiments according to the present invention, the device is an intravenous needle having the polymer and prodrug (for instance, a prodrug of an anticoagulant such as heparin or codrug thereof) coated thereon.

As discussed above, a device according to the present invention comprises a polymer that is bioerodible or non-bioerodible. The choice of bioerodible versus non-bioerodible polymer is made based upon the intended end use of the system or device. In some embodiments according to the present invention, the polymer is advantageously bioerodible. For instance, where the system is a coating on a surgically implantable device, such as a screw, stent, pacemaker, etc., the polymer is advantageously bioerodible. Other embodiments according to the present invention in which the polymer is advantageously bioerodible include devices that are implantable, inhalable, or injectable suspensions or dispersions of prodrug in a polymer, wherein the further elements (such as screws or anchors) are not utilized.

In some embodiments according to the present invention wherein the polymer is poorly permeable and bioerodible, the rate of bioerosion of the polymer is advantageously sufficiently slower than the rate of drug release so that the polymer remains in place for a substantial period of time after the drug has been released, but is eventually bioeroded and resorbed into the surrounding tissue. For example, where the device is a bioerodible suture comprising the drug suspended or dispersed in a bioerodible polymer, the rate of bioerosion of the polymer is advantageously slow enough that the drug is released in a linear manner over a period of about three to about 14 days, but the sutures persist for a period of about three weeks to about six months. Similar devices according to the present invention include surgical staples comprising a prodrug suspended or dispersed in a bioerodible polymer.

In other embodiments according to the present invention, the rate of bioerosion of the polymer is advantageously on the same order as the rate of drug release. For instance, where the system comprises a prodrug suspended or dispersed in a polymer that is coated onto a surgical implement, such as an orthopedic screw, a stent, a pacemaker, or a non-bioerodible suture, the polymer advantageously bioerodes at such a rate that the surface area of the prodrug that is directly exposed to the surrounding body tissue remains substantially constant over time.

In some embodiments according to the present invention, the polymer is non-bioerodible, or is bioerodible only at a rate slower than a dissolution rate of the low-solubility pharmaceutical prodrug, and the diameter of the granules is such that when the coating is applied to the stent, the granules' surfaces are exposed to the ambient tissue. In such embodiments, dissolution of the low-solubility pharmaceutical prodrug is proportional to the exposed surface area of the granules.

In other embodiments according to the present invention, the polymer vehicle is permeable to water in the surrounding tissue, e.g. in blood plasma. In such cases, water solution may permeate the polymer, thereby contacting the low-solubility pharmaceutical prodrug. The rate of dissolution may be governed by a complex set of variables, such as the polymer's permeability, the solubility of the low-solubility pharmaceutical prodrug, the pH, ionic strength, and protein composition, etc. of the physiologic fluid. In certain embodiments, however the permeability may be adjusted so that the rate of dissolution is governed primarily, or in some cases practically entirely, by the solubility of the low-solubility pharmaceutical prodrug in the ambient liquid phase.

In some embodiments according to the present invention, the polymer is non-bioerodible. Non-bioerodible polymers are especially useful where the system includes a polymer intended to be coated onto, or form a constituent part, of a surgical implement that is adapted to be permanently, or semi-permanently, inserted or implanted into a body. Exemplary devices in which the polymer advantageously forms a permanent coating on a surgical implement include an orthopedic screw, a stent, a prosthetic joint, an artificial valve, a permanent suture, a pacemaker, etc.

A surgical system according to the present invention is used in a manner suitable for the desired therapeutic effect. For instance in some embodiments according to the invention, the mode of administration is advantageously by injection. In such cases, the system is a liquid, and is introduced into the desired locus by taking the system up into the barrel of a syringe and injecting the system through a needle into the desired locus. Such a mode of administration is suitable for intramuscular injection, for instance intramuscular injection of sustained-release formulations of microbicides, including antibiotics, antivirals, and steroids. This mode of administration is also useful where the desired therapeutic effect is the sustained release of hormones such as thyroid medication, birth control prodrugs, estrogen for estrogen therapy, etc. The skilled clinician will appreciate that this mode of administration is adaptable to various therapeutic milieus, and will adapt the particular polymer and drug of the system to the desired therapeutic effect.

In embodiments according to the present invention in which the mode of administration is to be by injection, the system is advantageously a relatively non-polar drug suspended or dispersed in a viscous polymer vehicle. The system is, in such cases, a stable suspension or dispersion of non-polar drug in liquid polymer vehicle. Advantageously, the polymer vehicle will be either non-bioerodible or will bioerode at a rate slower than the rate of diffusion of the drug into the surrounding tissue. In such cases, the system stays in place in place relative to the surrounding tissue, preventing the drug from being prematurely released into the surrounding tissue.

In other embodiments according to the present invention, the system is a relatively non-polar liquid suspended or dispersed in a liquid polymer. In such cases, the system further comprises an emulsifier that maintains the relatively non-polar drug in a stable, dispersed, state within the polymer. The polymer vehicle advantageously is non-bioerodible, or is bioerodible at a slower rate than the rate of diffusion of the drug, so that the system maintains the location of the drug relative to the surrounding tissue over the full period of drug release.

The precise properties of the system according to the present invention depend upon the therapeutic use intended, the physical state of the drug to incorporated into the system under physiologic conditions, etc.

In some embodiments according to the present invention, the system according to the present invention is advantageously a solid device of a shape and form suitable for implantation, for instance subcutaneously, etc. In some embodiments according to the present invention, the system is in the shape of an elongated ovoid, the prodrug is of a non-polar drug, such as a hormone, and the polymer is a solid polymer whose permeability is such that it is not the primary rate-determining factor in the rate of release of the drug. In particular embodiments according to the present invention, the polymer is bioerodible. In other embodiments according to the present invention, the polymer is non-bioerodible.

In embodiments according to the present invention wherein the device comprises a substrate and a coating on the substrate, such as a screw, stent, pacemaker, prosthetic joint, etc., the device is used in substantially the manner of the corresponding prior art surgical implement. For instance, a device according to the present invention that comprises a screw coated with a composition comprising a low solubility prodrug, such as an antibiotic or FU-naproxen, suspended or dispersed in a polymer, is screwed into a bone in the same manner as a prior art screw. The screw according to the present invention then releases drug, in a sustained time-wise fashion, thereby conferring therapeutic benefits, such as antibiotic, anti-inflammatory, and antiviral effects, to the tissue surrounding the device, such as muscle, bone, blood, etc.

As used in this specification and the appended claims, sustained release means release via rate kinetics such that the permeability of the polymer is non-rate limiting with respect to the rate of release of the drug.

In embodiments according to the present invention wherein the device is a surgical implement into which the prodrug and polymer have been incorporated as a constituent part, the polymer is advantageously a solid having physical properties appropriate for the particular application of the device. For instance, where the device is a suture, the polymer will have strength and bioerodibility properties suitable for the particular surgical situation. Where the device is a screw, stent, etc, the polymer is advantageously a rigid solid forming at least part of the surgical implement. In particular embodiments according to the present invention, such as where the system is part of a prosthetic joint, the polymer advantageously is non-bioerodible and remains in place after the prodrug has been released into the surrounding tissue. In other embodiments according to the present invention, such as in the case of bioerodible sutures, the polymer bioerodes after release of substantially all the prodrug.

While exemplary embodiments of the invention will be described with respect to the treatment of restenosis and related complications following percutaneous transluminal coronary angioplasty, it is important to note that the local delivery of drug/drug combinations may be utilized to treat a wide variety of conditions utilizing any number of medical devices, or to enhance the function and/or life of the device. For example, intraocular lenses, placed to restore vision after cataract surgery is often compromised by the formation of a secondary cataract. The latter is often a result of cellular overgrowth on the lens surface and can be potentially minimized by combining a drug or drugs with the device. Other medical devices which often fail due to tissue in-growth or accumulation of proteinaceous material in, on and around the device, such as shunts for hydrocephalus, dialysis grafts, colostomy bag attachment devices, ear drainage tubes, leads for pace makers and implantable defibrillators can also benefit from the device-drug combination approach.

Devices which serve to improve the structure and function of tissue or organ may also show benefits when combined with the appropriate prodrug or codrugs. For example, improved osteointegration of orthopedic devices to enhance stabilization of the implanted device could potentially be achieved by combining it with prodrugs such as bone morphogenic protein. Similarly other surgical devices, sutures, staples, anastomosis devices, vertebral disks, bone pins, suture anchors, hemostatic barriers, clamps, screws, plates, clips, vascular implants, tissue adhesives and sealants, tissue scaffolds, various types of dressings, bone substitutes, intraluminal devices, and vascular supports could also provide enhanced patient benefit using this drug-device combination approach. Essentially, any type of medical device may be coated in some fashion with a prodrug or codrug which enhances treatment over use of the singular use of the device or pharmaceutical prodrug.

The subject devices can be used to deliver such pharmaceutical drugs as: antiproliferative/antimitotic prodrugs including natural products such as vinca alkaloids (i.e. vinblastine, vincristine, and vinorelbine), paclitaxel, epidipodophyllotoxins (i.e. etoposide, teniposide), antibiotics (dactinomycin (actinomycin D) daunorubicin, doxorubicin and idarubicin), anthracyclines, mitoxantrone, bleomycins, plicamycin (mithramycin) and mitomycin, enzymes (L-asparaginase which systemically metabolizes L-asparagine and deprives cells which do not have the capacity to synthesize their own asparagine); antiplatelet prodrugs; antiproliferative/antimitotic alkylating prodrugs such as nitrogen mustards (mechlorethamine, cyclophosphamide and analogs, melphalan, chlorambucil), ethylenimines and methylmelamines (hexamethylmelamine and thiotepa), alkyl sulfonates-busulfan, nirtosoureas (carmustine (BCNU) and analogs, streptozocin), trazenes - dacarbazinine (DTIC); antiproliferative/antimitotic antimetabolites such as folic acid analogs (methotrexate), pyrimidine analogs (fluorouracil, floxuridine, and cytarabine), purine analogs and related inhibitors (mercaptopurine, thioguanine, pentostatin and 2-chlorodeoxyadenosine fcladribinel); platinum coordination complexes (cisplatin, carboplatin), procarbazine, hydroxyurea, mitotane, aminoglutethimide; hormones (i.e. estrogen); anticoagulants (heparin, synthetic heparin salts and other inhibitors of thrombin); fibrinolytic prodrugs (such as tissue plasminogen activator, streptokinase and urokinase), aspirin, dipyridamole, ticlopidine, clopidogrel, abciximab; antimigratory; antisecretory (breveldin); antiinflammatory: such as adrenocortical steroids (cortisol, cortisone, fludrocortisone, prednisone, prednisolone, 6U-methylprednisolone, triamcinolone, betamethasone, and dexamethasone), non-steroidal prodrugs (salicylic acid derivatives i.e. aspirin; para-aminophenol derivatives i.e. acetominophen; indole and indene acetic acids (indomethacin, sulindact and etodalac), heteroaryl acetic acids (tolmetin, diclofenac, and ketorolac), arylpropionic acids (ibuprofen and derivatives), anthranilic acids (mefenamic acid, and meclofenamic acid), enolic acids (piroxicam, tenoxicam, phenylbutazone, and oxyphenthatrazone), nabumetone, gold compounds (auranofin, aurothioglucose, gold sodium thiomalate); immunosuppressives (cyclosporine, tacrolimus (FK-506), sirolimus (rapamycin), azathioprine, mycophenolate mofetil); angiogenic prodrugs: vascular endothelial growth factor (VEGF), fibroblast growth factor (FGF); angiotensin receptor blocker; nitric oxide donors; anti-sense oligionucleotides and combinations thereof; cell cycle inhibitors, mTOR inhibitors, and growth factor signal transduction kinase inhibitors.

In certain embodiments, the prodrug is formed using an opiod. Exemplary opioids include morophine derivatives, such as apomorphine, buprenorphine, codeine, dihydrocodeine, dihydroetorphine, diprenorphine, etorphine, hydrocodone, hydromorphone, levorphanol, meperidine, metopon, o-methylnaltrexone, morphine, naloxone, naltrexone, normorphine, oxycodone, and oxymorphone. In other embodiments, the opiod is a fentanyl derivative which can be deritized to form the prodrug, such as β-hydroxy-3-methylfentanyl.

As used in regard to the low-solubility pharmaceutical prodrug, the term "low-solubility" relates to the solubility of the pharmaceutical prodrug in biological fluids, such as blood plasma, lymphatic fluid, peritoneal fluid, etc. In general, "low-solubility" means that the pharmaceutical prodrug is only very slightly soluble in aqueous solutions having pH in the range of about 5 to about 8, and in particular to physiologic solutions, such as blood, blood plasma, etc. Some low-solubility prodrugs according to the present invention will have solubilities of less than about 1 mg/ml, less than about 100 µg/ml, preferably less than about 20 µg/ml, more preferably less than about 15 µg/ml, and more preferably less than about 10 µg/ml. Solubility is in water at a temperature of 25°C as measured by the procedures set forth in the 1995 USP, unless otherwise stated. This includes compounds which are slightly soluble (about 10mg/ml to about 1 mg/ml), very slightly soluble (about 1 mg/ml to about 0.1 mg/ml) and practically insoluble or insoluble compounds (less than about 0.01 mg/ml).

Suitable prodrugs useful in the present invention include prodrugs of immune response modifiers such as cyclosporin A and FK 506, corticosteroids such as dexamethasone and triamcinolone acetonide, angiostatic steroids such as trihydroxy steroids, antiparasitic prodrugs such as atovaquone, anti-glaucoma prodrugs such as ethacrynic acid, antibiotics including ciprofloxacin, differentiation modulators such as retinoids (e.g., trans-retinoic acid, cis-retinoic acid and analogues), antiviral prodrugs including high molecular weight low (10-mers), anti-sense compounds, anticancer prodrugs such as BCNU, non-steroidal anti-inflammatory prodrugs such as indomethacin and flurbiprofen, and prodrugs comprising a conjugate of at least two compounds linked via a reversible covalent or ionic bond that is cleaved at a desired site in a body to regenerate an active form of each compound. In embodiments of the present invention, the prodrug is relatively insoluble in aqueous media, including physiological fluids, such as blood serum, mucous, peritoneal fluid, limbic fluid, etc. In still further embodiments according to the present invention, suitable prodrugs include drugs, which are lipophilic derivatives of hydrophilic drugs, that are easily converted into their hydrophilic drugs under physiological accessible conditions. Reference may be made to any standard pharmaceutical textbook for the procedures to obtain a low solubility form of a drug. In this regard, the present invention is especially suitable for prodrugs that heretofore have not found broad application due to their inherent low solubility, or have found only limited application in oil-based or other lipid-based delivery vehicles. In certain embodiments, the present invention provides an intraluminal medical device for implantation into a lumen of a blood vessel, in particular adjacent an intraluminal lesion such as an atherosclerotic lesion, for maintaining patency of the vessel. In particular the present invention provides an elongate radially expandable tubular stent having an interior luminal surface and an opposite exterior surface extending along a longitudinal stent axis, the stent having a coating on at least a portion of the interior or exterior surface thereof. The local delivery of drug combinations from a stent has the following advantages; namely, the prevention of vessel recoil and remodeling through the scaffolding action of the stent and the prevention of multiple components of neointimal hyperplasia or restenosis as well as a reduction in inflammation and thrombosis. This local administration of drugs to stented coronary arteries may also have additional therapeutic benefit. For example, higher tissue concentrations of the drugs may be achieved utilizing local delivery, rather than systemic administration. In addition, reduced systemic toxicity may be achieved utilizing local delivery rather than systemic administration while maintaining higher tissue concentrations. Also in utilizing local delivery from a stent rather than systemic administration, a single procedure may suffice with better patient compliance. An additional benefit of combination drug therapy may be to reduce the dose of each of the therapeutic drugs, prodrugs or compounds, thereby limiting their toxicity, while still achieving a reduction in restenosis, inflammation and thrombosis. Local stent-based therapy is therefore a means of improving the therapeutic ratio (efficacy/toxicity) of anti-restenosis, anti-inflammatory, antithrombotic drugs, prodrugs or compounds.

There are a multiplicity of different stents that may be utilized following percutaneous transluminal coronary angioplasty. Although any number of stents may be utilized in accordance with the present invention, for simplicity, a limited number of stents will be described in exemplary embodiments of the present invention. The skilled artisan will recognize that any number of stents may be utilized in connection with the present invention. In addition, as stated above, other medical devices may be utilized.

A stent is commonly used as a tubular structure left inside the lumen of a duct to relieve an obstruction. Commonly, stents are inserted into the lumen in a non-expanded form and are then expanded autonomously, or with the aid of a second device in situ. A typical method of expansion occurs through the use of a catheter-mounted angioplasty balloon which is inflated within the stenosed vessel or body passageway in order to shear and disrupt the obstructions associated with the wall components of the vessel and to obtain an enlarged lumen.

The stents of the present invention may be fabricated utilizing any number of methods. For example, the stent may be fabricated from a hollow or formed stainless steel tube that may be machined using lasers, electric discharge milling, chemical etching or other means. The stent is inserted into the body and placed at the desired site in an unexpanded form. In one exemplary embodiment, expansion may be effected in a blood vessel by a balloon catheter, where the final diameter of the stent is a function of the diameter of the balloon catheter used.

It should be appreciated that a stent in accordance with the present invention may be embodied in a shape-memory material, including, for example, an appropriate alloy of nickel and titanium or stainless steel.

Structures formed from stainless steel may be made self-expanding by configuring the stainless steel in a predetermined manner, for example, by twisting it into a braided configuration. In this embodiment after the stent has been formed it may be compressed so as to occupy a space sufficiently small as to permit its insertion in a blood vessel or other tissue by insertion means, wherein the insertion means include a suitable catheter, or flexible rod.

On emerging from the catheter, the stent may be configured to expand into the desired configuration where the expansion is automatic or triggered by a change in pressure, temperature or electrical stimulation.

Regardless of the design of the stent, it is preferable to have the drug combination dosage applied with enough specificity and a sufficient concentration to provide an effective dosage in the lesion area. In this regard, the "reservoir size" in the coating is preferably sized to adequately apply the drug combination dosage at the desired location and in the desired amount.

In an alternate exemplary embodiment, the entire inner and outer surface of the stent may be coated with drug/drug combinations in therapeutic dosage amounts. It is, however, important to note that the coating techniques may vary depending on the drug combinations. Also, the coating techniques may vary depending on the material comprising the stent or other intraluminal medical device.

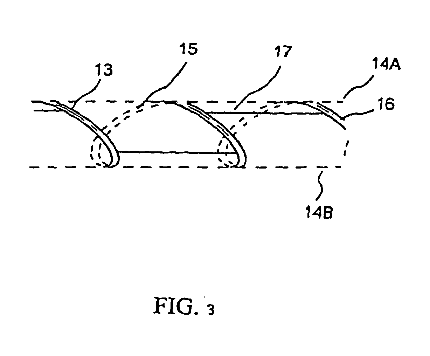

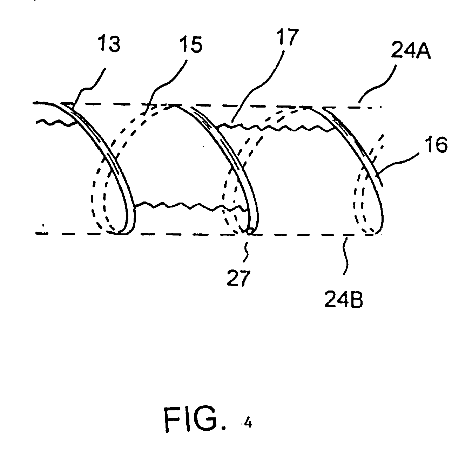

An embodiment of an intraluminal device (stent) according to the present invention is depicted in FIGs. 3 and 4.

FIG. 3 shows a side plan view of a preferred elongate radially expandable tubular stent 13 having a surface coated with a sustained release drug delivery system in a non-deployed state. As shown in FIG. 3, the stent 13 has its radially outer boundaries 14A, 14B at a non-deployed state. The interior luminal surface 15, the exterior surface 16, or an entire surface of the stent 13 may be coated with a sustained release drug delivery system or comprise a sustained release drug delivery system. The interior luminal surface 15 is to contact a body fluid, such as blood in a vascular stenting procedure, while the exterior surface 16 is to contact tissue when the stent 13 is deployed to support and enlarge the biological vessel or duct.

In an alternate embodiment, an optional reinforcing wire 17 that connects two or more of the adjacent members or loops of the stent structure 13 is used to lock-in and/or maintain the stent at its expanded state when a stent is deployed. This reinforcing wire 17 may be made of a Nitinol or other high-strength material. A Nitinol device is well known to have a preshape and a transition temperature for said Nitinol device to revert to its preshape. One method for treating an intraluminal tissue of a patient using a surface coated stent 13 of the present invention comprises collapsing the radially expandable tubular stent and retracting the collapsed stent from a body of a patient. The operation for collapsing a radially expandable tubular stent may be accomplished by elevating the temperature so that the reinforcing wire 17 is reversed to its straightened state or other appropriate state to cause the stent 13 to collapse for removing said stent from the body of a patient.

FIG. 4 shows an overall view of an elongate radially expandable tubular stent 13 having a sustained release drug delivery system coated stent surface at a deployed state. As shown in FIG. 4, the stent 13 has its radially outer boundaries 24A, 24B at a deployed state. The interior luminal surface 14, the exterior surface 16, or an entire surface of the stent 13 may be coated or may comprise the sustained release drug delivery system. The interior luminal surface 15 is to contact a body fluid, such as blood in a vascular stenting procedure, while the exterior surface16 is to contact tissue when the stent 13 is deployed to support and enlarge the biological vessel. The reinforcing wire 17 may be used to maintain the expanded stent at its expanded state as a permanent stent or as a temporary stent. In the case of the surface coated stent 13 functioning as a temporary stent, the reinforcing wire 17 may have the capability to cause collapsing of the expanded stent.

The deployment of a stent can be accomplished by a balloon on a delivery catheter or by self-expanding after a pre-stressed stent is released from a delivery catheter. Delivery catheters and methods for deployment of stents are well known to one who is skilled in the art. The expandable stent 13 may be a self-expandable stent, a balloon-expandable stent, or an expandable-retractable stent. The expandable stent may be made of memory coil, mesh material, and the like.

### III. Examples

The present invention can be more fully understood with reference to the following examples.

Prodrug TC-112 comprising a conjugate of 5-fluorouracil and naproxen linked via a reversible covalent bond, and prodrug G.531.1 comprising a conjugate of 5-fluorouracil and fluocinolone acetonide were prepared in accordance with the methods set forth in U.S. Patent No. 6,051,576. The structure of these compounds is reproduced below.

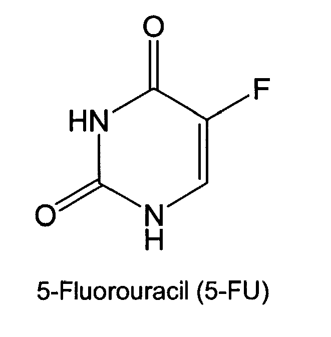

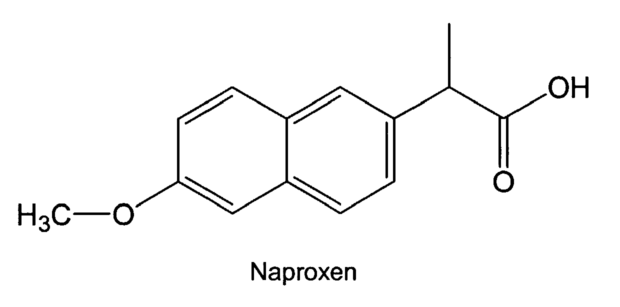

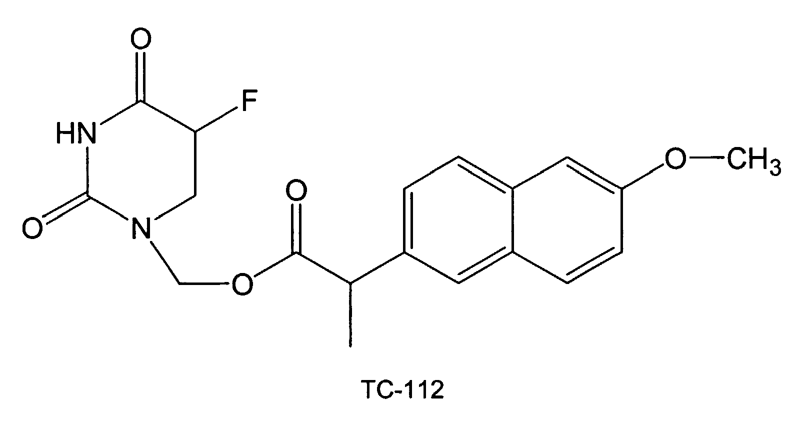

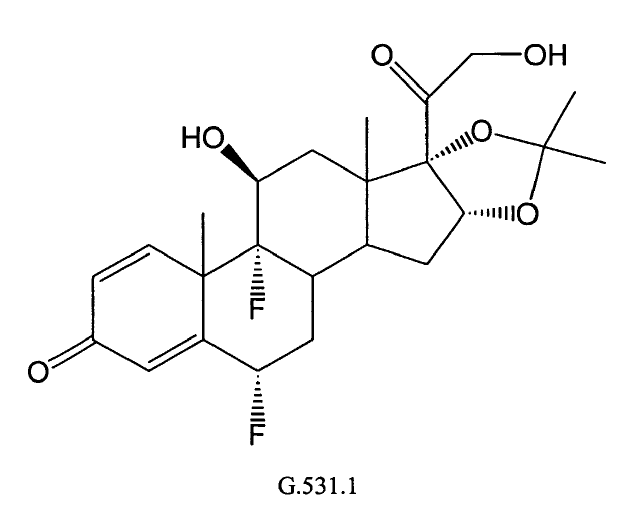

The following examples are intended to be illustrative of the disclosed invention. The examples are non-limiting, and the skilled artisan will recognize that other embodiments are within the scope of the disclosed invention.

### Example 1

To 20 gm of 10% (w/v) aqueous poly(vinyl alcohol) (PVA) solution, 80.5 mg of prodrug TC-112 was dispersed. 5 pieces of glass plates were then dipping coated with this TC-112/PVA suspension and followed by air-drying. The coating and air-drying was repeated four more times. At the end about 100 mg of TC-112/PVA was coated on each glass plates. The coated glass plates were then heat treated at 135°C for 5 hours. After cooling to room temperature, the glass plates were individually placed in 20 ml of 0.1 M mol phosphate buffer (pH 7.4, 37 °C) for release test. Sample was taken daily and entire release media were replaced with fresh one at each sampling time. The drugs and TC-112 released in the media were determined by reverse-phase HPLC. The half-life for TC-112 in pH 7.4 buffer is 456 min, in serum is 14 min.

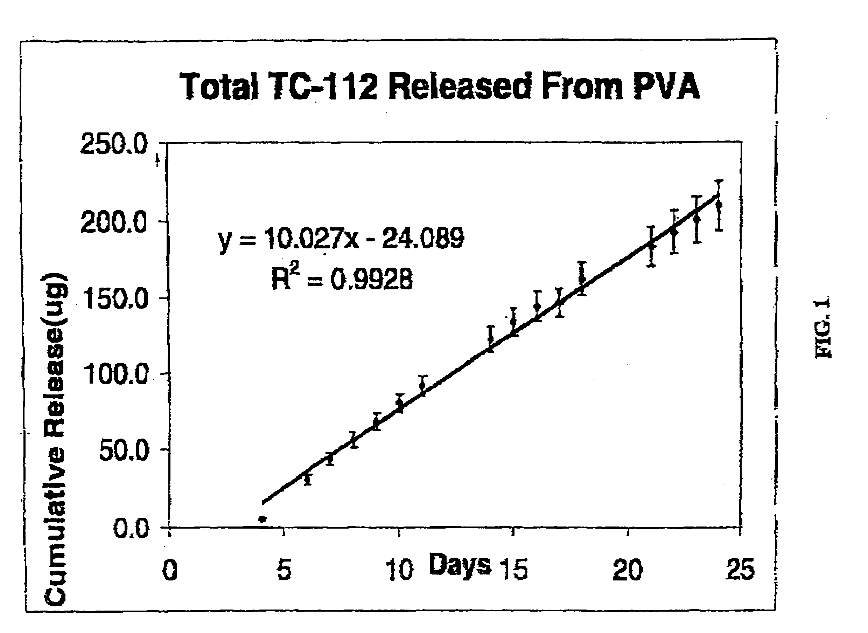

The results are shown in FIG. 1, which shows the total cumulative release of TC-112 from PVA coated glass plates. The slope of the curve demonstrates that TC-112 is released at 10 µg/day. The data represent both intact and constituents of the compound TC-112.

### Example 2

12.0 gm of silicone part A (Med-6810A) were mixed with 1.2 gm of silicone part B (Med-6810B), and degassed in sonicator for 10 min, followed by water aspirator. 41.2 mg of (TC-112) were dispersed in this degassed silicone, and degassed again. 0.2 gm of the mixture was spread on one surface of a glass plate. The glass plates (total 5) were then placed in oven and heated at 105 °C for 20 min. to cure. After removing from the oven and cooled to room temperature, 0.2 gm of the mixture was spread on the other uncoated surface of each glass plate. The coated glass plates were then heat treated again at 105 °C for 20 min. After cooling to room temperature, the glass plates were individually placed in 20 ml of 0.1 M phosphate buffer (pH 7.4, 37 °C) for release test. Samples were taken daily, and the entire release media was replaced with fresh media at each sampling time. The drugs (5FU and TA) and TC-112 released in the media were determined by HPLC.

The total TC-112 release for silicone coating was calculated as follows. The molecular weight of Naproxen is 230.3, and the molecular weight for 5-Fluorouracil is 130.1, while the inventive compound (TC-112) generated from these two drugs has a molecular weight of 372.4. To detect x mg of naproxen, this means that x*372.4/230.3 mg of TC-112 was hydrolyzed. The total TC-112 released equals the sum of TC-112 detected in the release media and the TC-112 hydrolyzed. For example, up to day 6, 43.9 mg of naproxen is detected, this means 71.0 (43.9*372.4/230.3) mg of TC-112 was hydrolyzed, at the same time, 51.4 mg of TC-112 is detected in buffer, therefore a total of 122.4mg (51.4 plus 71.0) of TC-112 is released up to day 6.

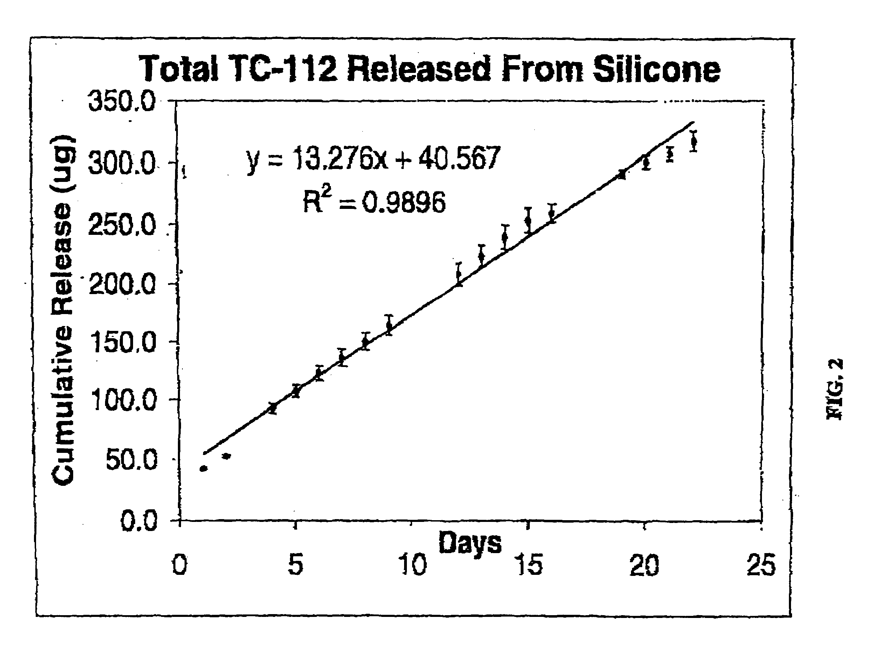

The results are shown in FIG. 2, which shows the total cumulative release of TC-112 from silicone coated glass plates. The slope of the curve demonstrates that TC-112 is released at 13.3 µg/day. Again, the data represent both intact and constituents of the inventive compound. The similarity in the slopes demonstrates that the polymers have little effect on the release of the drug.

### Example 3

A mixture of 3.3 gm Chronoflex C(65D) (Lot# CTB-G25B-1234) dispersion containing 0.3 gm of Chronoflex C(65D) and 2.2 gm Chronoflex C(55D) (Lot# CTB-121B-1265) dispersion containing 0.2 gm of Chronoflex C (55D), both in dimethyl acetamide (DMAC) (1:10, w/w) was prepared by mixing the two dispersions together. To this mixture, 6.0 gm of tetrahydrofurane (HPLC grade) were added and mixed. The final mixture was not a clear solution. Then 101.5 mg of a co-drug of 5-fluorouracil (5FU) and triamcinolone acetonide (TA) (the co-drug being defined as "TC-32") was added and dissolved into the polymer solution.

Ten (10) HPLC inserts were then coated with the polymer/TC-32 solution by dipping, which was then followed by air-drying under ambient temperature. The coating and air-drying process was repeated four (4) times (5 times total) until a total of about 10 mg of polymer/TC-32 was applied to each insert. The inserts were then placed in an oven at 80°C for two hour to remove the residue of the solvent.

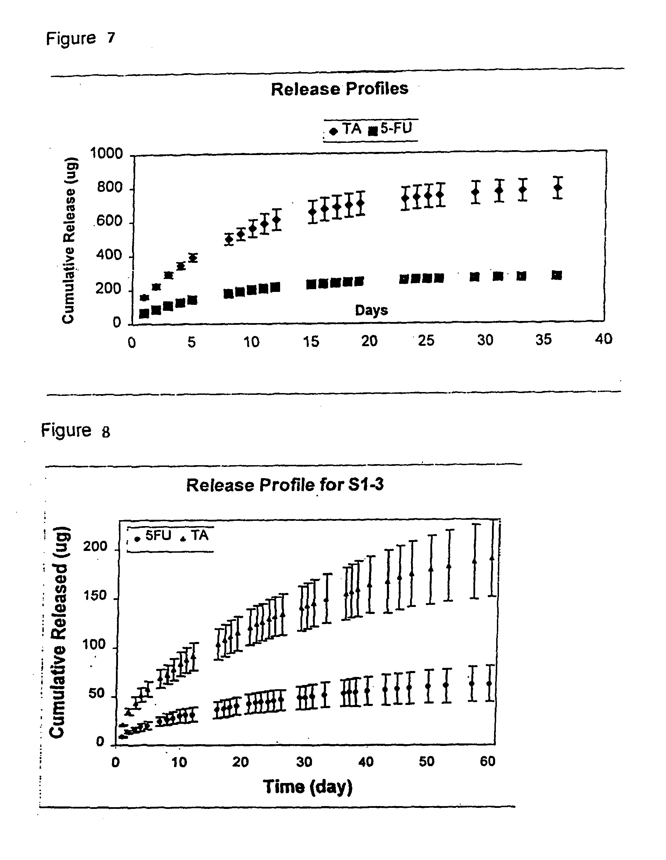

The inserts were placed individually in 20 ml of 0.1 m phosphate buffer, pH 7.4, in glass tube and monitoring of the release of compounds from the inserts at 37°C was begun. Samples were taken daily, and the entire media was replaced with fresh media at each sampling time. The drugs released in the media were determined by HPLC. Because of the short half-life of TC-32 in buffer, no TC-32 was detectable in the release media; only amounts of parent drugs, 5-FU and TA, could be determined. The release profiles are displayed in Figure 7.

### Example 4

To 5.0 gm of stirred dimethyl acetamide (DMAC), 300 mg of Chronoflex C(65D) (Lot# CTB-G25B-1234) and 200 mg of Chronoflex C(55D) (Lot# CTB-121B-1265) were added. The polymer was slowly dissolved in DMAC (about 4 hours). Then 5.0gm of THF was added to the polymer dispersion. The mixture was not a clear solution. Then 100.9 mg of TC-32 was added and dissolved in the mixture.

Three (3) Stents, supplied by Guidant Corp, were coated then with the polymer/TC-32 solution by dipping and followed by air-drying under ambient temperature. The coating and air-drying process was repeated a few times till a total of about 2.0 mg of polymer/TC-32 were applied to each stent. The coated stents were air-dried under ambient temperature in a biological safety cabinet over night. The stents were then vacuum dried at 80°C for two hour to remove the residue of the solvent. Afterwards they were placed individually in 5.0 ml of 0.1 m phosphate buffer, pH 7.4, in glass tube and monitoring of the release of compounds from the stents was at 37°C was begun. Samples were taken daily, and the entire media was replaced with a fresh one at each sampling time. The drugs released in the media were determined by HPLC. The release profiles were shown in the Figure 8. No TC-32 was detectable in the release media.

### Example 5

Polyurethane (PU) was first dissolved in tetrahydrofuran. Into this solution bioreversible conjugates of 5-FU and TA were dissolved and the resulting solution spray coated onto coronary Tetra stents produced by Guidant. After air-drying, the stents were vacuum dried at 50 °C for 2 hours to remove solvent residue, and subjected to plasma treatment and gamma-irradiation. Two different levels of drug loading were applied to stents: 80 ug Low Dose (13%) and 600 ug High Dose (60%). The release rate was determined in vitro by placing the coated stents (expanded) in 0.1M phosphate buffer (pH 7.4) at 37°C. Samples of the buffer solution were periodically removed for analysis by HPLC, and the buffer was replaced to avoid any saturation effects.

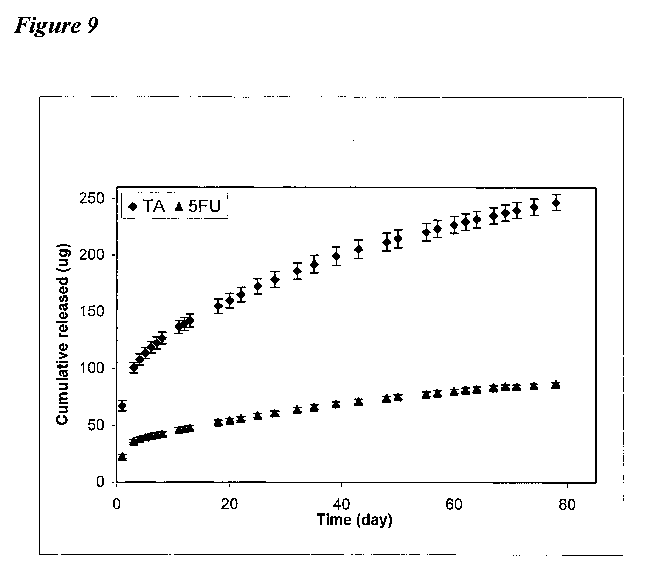

The results shown in Figure 9 illustrate the release pattern in vitro for a High Dose coated stent. The pattern followed a pseudo logarithmic pattern with approximately 70% being released in 10 weeks. A similar pattern is seen in both High Dose and Low Dose loaded stents. TA and 5-FU were released in an equimolar fashion at all times during the experiments. No co-drugs of 5-FU/TA were detectable in the release media.

### Example 6

Polyurethane (1.008 gm) was added to 50.0 gm of tetrahydrofuran (THF). The mixture was stirred overnight to dissolve the polymer. 5.0 gm of the polymer solution was diluted with 10.0 gm of THF. 150.2 mg of a co-drug of 5-fluorouracil (5FU) and triamcinolone acetonide (TA) (the co-drug being defined as "TC-32") was added to the polymer solution and dissolved. The coating solution was prepared with 60% codrug loading. A 13% codrug loaded coating solution was also prepared. Bare stents (Tetra, Guidant, Lot# 1092154, 13mm Tetra) were washed with isopropanol, air-dried, and spray coated with the coating solution using a precision airbrush. The coating was repeated until approximately 1.0 mg of total coating had been applied to each stent. The coated stents were vacuum dried for two hours at 50°C to remove solvent residue, then subjected to plasma treatment and gamma-irradiation.

Co-drug coated stents were test in two groups. Group One stents were placed individually into a glass tube containing 5.0 ml of 0.1 M phosphate buffer (pH 7.4). Samples were taken periodically and the concentration of co-drug in the buffer was tested by HPLC. The entire release media was replaced after each sample.

Group Two stents were placed in vivo. Three common swine had TC-32 coated stents implanted into the left anterior descending (LAD) coronary artery on study day 1. The stents were harvested on study day 5 and then placed in 0.1 M phosphate buffer as describe for Group One stents. The amount of each drug released into the media was determined by HPLC. The intact codrug was not detectable in release media.

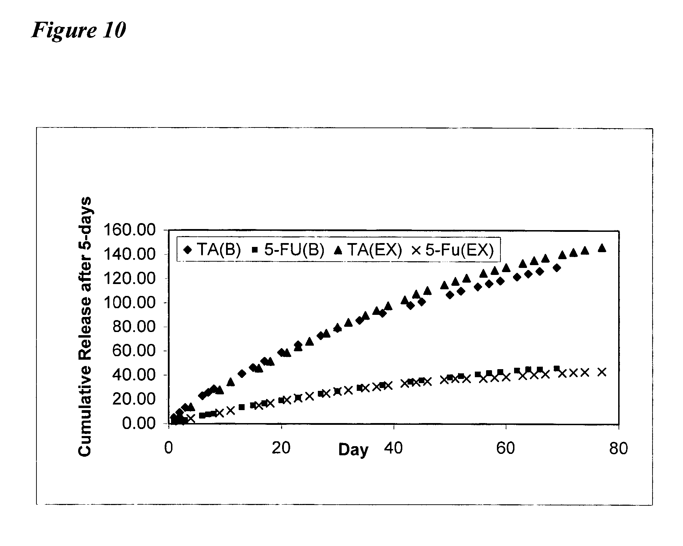

The results are shown in Fig. 10, showing the comparative drug release profiles between explanted stents and non-implanted stents. The release patterns for both explanted and pre-implanted stents indicate that in-vivo release may be predicted by in vitro release patterns.

### Example 7

Fourteen (14) domestic swine received a maximum of three (3) stents deployed in any of the three-epicardial coronaries (LAD, LCX, and RCA). Some animals were given only control stents, comprising either Bare Metal Tetra Coronary Stent on Cross Sail Rx balloon delivery system (Control), or PU Coated Tetra Coronary Stent on Cross Sail Rx balloon delivery system (Control). Other animals were given drug-coated stents either in Low Dose (80µg TA+5FU (13%)) or High Dose (600µg TA+5FU (60%)). The stents were implanted into arteries of the animals. Each stent was advanced to the desired location in the artery, and was deployed using an inflation device. The pressure of the inflation device was chosen to achieve a balloon to artery ratio of 1.1-1.2:1.

After 28 days, arterial sections directly adjacent to the stents were surgically excised and embedded in a methacrylate resin. Histologic 5-µm sections were cut and stained with Verhoeffs elastin and Hematoxylin and Eosin stains, and the thickness of each excised section was measured. The results are shown in the table below for both High and Low Dose drug-coated stents. The response at 28 days in both low-dose and high-dose experimental groups shows a profound reduction in intimal thickness attributed to the co-release of TA and 5FU from polymer coated Tetra stents.

| | **Bare Metal** | **Polymer** | **Low Dose** | **High Dose** |
|---|---|---|---|---|
| **Balloon: artery ratio** | 1.07±0.05 | 1.11±0.07 | 1.13±0.05 | 1.11±0.08 |
| **Intimal Thickness (mm)** | 0.29±0.03 | 0.36±0.08 | 0.13±0.01^{ξ} | 0.13±0.04^{ϑ} |
| **Medial area (mm²)** | 1.39±0.10 | 1.98±0.41 | 0.96±0.06^{§} | 0.98:t0.07^{ζ} |

| | | | | |
|---|---|---|---|---|
| ξ p=0.0008 Bare Metal vs. Low Dose, p=0.03 Polymer vs. Low Dose § p=0.002 Bare Metal vs. Low Dose, p=0.04 Polymer vs. Low Dose 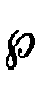 p=0.02 Bare Metal vs. High Dose, p=0.07 Polymer vs. High Dose ζ p=0.01 Bare Metal vs. High Dose, p=0.07 Polymer vs. High Dose | | | | |

### Example 8

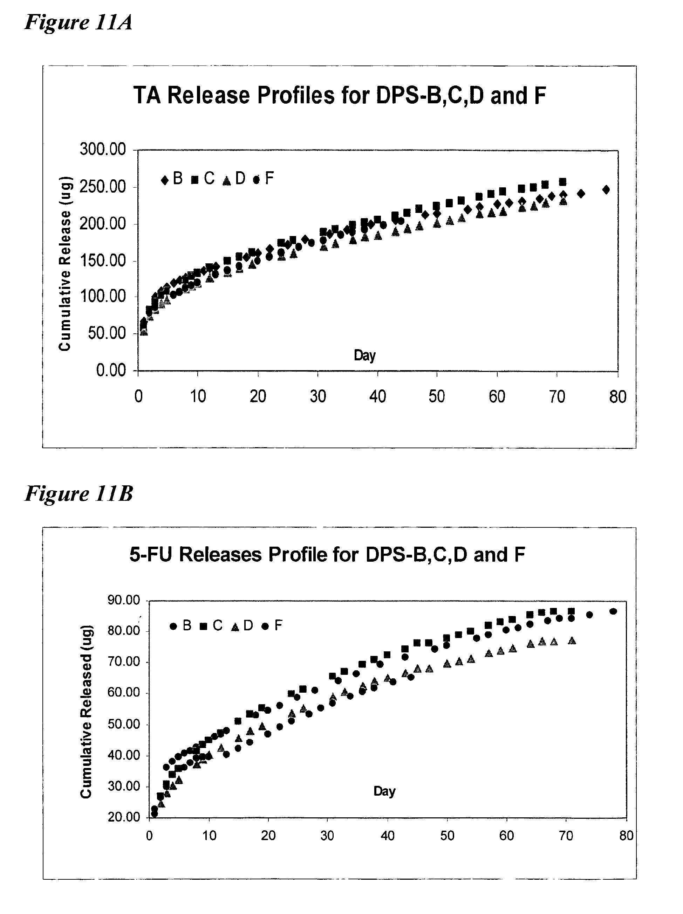

FIGS. 11A and 11B are graphs showing the effect of gamma irradiation and plasma treatment on drug release. Following plasma treatment and gamma-irradiation, the stents were inflated with a dilatation catheter (3.0 mm balloon size, 20mm long) and placed individually into a glass tube containing 5.0 ml of 0.1 M phosphate buffer (pH 7.4). Samples were taken periodically and the entire release media was replaced after each sample. The amount of each drug released into the media was determined by HPLC. The intact codrug was not detectable in release media.

### Example 9: Coating Example A

1.0gm of EMM (poly(ethyl acrylate and methyl methacrylate)copolymer), obtained by evaporating the Eudragit NE30D aqueous dispersion and air drying, was added in 9.0 gm acetone. To this dispersion, 51.5 mg of codrug of 5-Fluorouracil and Fluocinolone acetonide (G.531.1) were added and dissolved after stirring. By dipping them in the codrug/polymer solution, followed by air-drying, 10 HPLC inserts were coated with the codrug/polymer. The coating process was repeated several times until about 30 mg of codrug/polymer were coated on each of the glass tube. The coated inserts were then individually placed in 10.0 ml of 0.1 M phosphate buffer (pH 7.4, 37 °C) for release test. Sample was taken daily and entire release media were replaced with fresh media at each sampling time. The drugs and codrug released in the media were determined by HPLC.

### Example 10: Coating Example B

441.8 mg poly(ethylene-co-vinyl acetate) (EVA) is weighed and transferred to 15.0 ml of THF. The EVA is slowly swollen and then partly dissolved in the THF by ultrasonic and magnetic stirring. 88.2 mg of codrug (TC32) is added and dissolved into the polymer solution. 9 HPLC inserts are then coated with the polymer/codrug solution by dipping, followed by air-drying under ambient temperature. The coating and air-drying process is repeated a few times until a total of about 10 mg of polymer/codrug is applied to each insert. The inserts are then placed in oven at 50°C for one hour to remove the residue of the solvent. The weight and diameter of the inserts are checked before and after completion of the coating and recorded. The coated inserts were then individually placed in 10.0 ml of 0.1 M phosphate buffer (pH 7.4, 37 °C) for release test. Sample was taken daily and entire release media were replaced with fresh media at each sampling time. The drugs and codrug released in the media were determined by HPLC.

The purpose of the above description and examples is to illustrate some embodiments of the present invention without implying any limitation. It will be apparent to those of skill in the art that various modifications and variations may be made to the systems, devices and methods of the present invention without departing from the spirit or scope of the invention. All patents and publications cited herein are incorporated by reference in their entireties.

Further embodiments of the invention are disclosed in the clauses below.
a. A sustained release formulation comprising a polymer matrix and a prodrug, dispersed in the polymer, having a general formula of **A-L-B** in which
   A represents a drug moiety having a therapeutically active form for producing a clinical response in a patient;
   L represents a covalent linker linking A and B to form a prodrug, said linker being cleaved under physiological conditions to generate said therapeutically active form of A; and
   B represents a moiety which, when linked to A, results in the prodrug having a lower solubility than the therapeutically active form of A;
   wherein the solubility of therapeutically active form of A in water is greater than 1 mg/ml and the solubility of the prodrug in water is less than 1 mg/ml.
b. A sustained release formulation comprising a polymer matrix and a prodrug, dispersed in the polymer, having a general formula of **A::B** in which
   A represents a drug moiety having a therapeutically active form for producing a clinical response in a patient;
   :: represents a ionic bond between A and B that dissociates under physiological conditions to generate said therapeutically active form of A;
   B represents a moiety which, when ionically bonded to A, results in the prodrug having a lower solubility than the therapeutically active form of A; and
   wherein the solubility of therapeutically active form of A in water is greater than 1 mg/ml and the solubility of the prodrug in water is less than 1 mg/ml.
c. A sustained release formulation comprising a polymer matrix and a prodrug, dispersed in the polymer, having a general formula of **A-L-B** in which
   A represents a drug moiety having a therapeutically active form for producing a clinical response in a patient;
   L represents a covalent linker linking A and B to form a prodrug, said linker being cleaved under physiological conditions to generate said therapeutically active form of A; and
   B represents a moiety which, when linked to A, results in the prodrug having a lower solubility than the therapeutically active form of A;
   wherein, when disposed in biological fluid, said sustained release formulation provides sustained release of the therapeutically active form of A for a period of at least 24 hours, and, over the period of release, the concentration of the prodrug in fluid outside the polymer is less than 10% of the concentration of the therapeutically active form of A.
d. A sustained release formulation comprising a polymer matrix and a prodrug, dispersed in the polymer, having a general formula of **A::B** in which
   A represents a drug moiety having a therapeutically active form for producing a clinical response in a patient;
   :: represents a ionic bond between A and B that dissociates under physiological conditions to generate said therapeutically active form of A;
   B represents a moiety which, when ionically bonded to A, results in the prodrug having a lower solubility than the therapeutically active form of A; and
   wherein, when disposed in biological fluid, said sustained release formulation provides sustained release of the therapeutically active form of A for a period of at least 24 hours, and, over the period of release, the concentration of the prodrug in fluid outside the polymer is less than 10% of the concentration of the therapeutically active form of A.
e. A sustained release formulation comprising a polymer matrix and a prodrug, dispersed in the polymer, having a general formula of **A-L-B** in which
   A represents a drug moiety having a therapeutically active form for producing a clinical response in a patient;
   L represents a covalent linker linking A and B to form a prodrug, said linker being cleaved under physiological conditions to generate said therapeutically active form of A; and
   B represents a moiety which, when linked to A, results in the prodrug having a lower solubility than the therapeutically active form of A;
   wherein the therapeutically active form of A has a logP value at least 1 logP unit less than the logP value of the prodrug.
f. A sustained release formulation comprising a polymer matrix and a prodrug, dispersed in the polymer, having a general formula of **A::B** in which
   A represents a drug moiety having a therapeutically active form for producing a clinical response in a patient;
   :: represents a ionic bond between A and B that dissociates under physiological conditions to generate said therapeutically active form of A;
   B represents a moiety which, when ionically bonded to A, results in the prodrug having a lower solubility than the therapeutically active form of A; and
   wherein the therapeutically active form of A has a logP value at least 1 logP unit less than the logP value of the prodrug.
g. The sustained release formulation of clauses a or b, wherein the solubility of the prodrug is less than 100 µg/ml in water.
h. The sustained release formulation of any of clauses a-f, wherein B is a hydrophobic aliphatic moiety.
i. The sustained release formulation of any of clauses a-f, wherein B is a drug moiety having a therapeutically active form generated upon cleavage of said linker L or dissociation of said ionic bond.
j. The sustained release formulation of clause i, wherein A and B are the same drug moiety.
k. The sustained release formulation of clause i, wherein A and B are different drug moieties.
l.The sustained release formulation of any of clauses a-f, wherein B, after cleavage from the prodrug, is a biologically inert moiety.
m. The sustained release formulation of any of clauses a-f, wherein A is selected from immune response modifiers, anti-proliferatives, corticosteroids, angiostatic steroids, anti-parasitic drugs, anti-glaucoma drugs, antibiotics, anti-sense compounds, differentiation modulators, antiviral drugs, anticancer drugs, and non-steroidal anti-inflammatory drugs.
n. The sustained release formulation of clause i, wherein B is selected from immune response modifiers, anti-proliferatives, corticosteroids, angiostatic steroids, anti-parasitic drugs, anti-glaucoma drugs, antibiotics, anti-sense compounds, differentiation modulators, antiviral drugs, anticancer drugs, and non-steroidal anti-inflammatory drugs.
o. The sustained release formulation of any of clauses a-f, wherein the duration of release of the therapeutically active form of A from the polymer matrix is at least 24 hours.
p. The sustained release formulation of clause i, wherein A is 5-fluorouracil (5FU) and B is naproxen.
q. The sustained release formulation of any of clauses a-f or i, wherein at least one of A or B is an antineoplastic agent.
r. The sustained release formulation of clause q, wherein said antineoplastic agent selected from the group consisting of anthracyclines, vincaalkaloids, purine analogs, pyrimidine analogs, inhibitors of pyrimidine biosynthesis, and alkylating agents.
s. The sustained release formulation of clause q, wherein said antineoplastic drug is a fluorinated pyrimidine.
t. The sustained release formulation of clause q, wherein said antineoplastic drug is selected from the group consisting of 5-fluorouracil (5FU), 5'-deoxy-5-fluorouridine 5-fluorouridine, 2'-deoxy-5-fluorouridine, fluorocytosine, 5-trifluoromethyl-2'-deoxyuridine, arabinoxyl cytosine, cyclocytidine, 5-aza-2'-deoxycytidine, arabinosyl 5-azacytosine, 6-azacytidine, N-phosphonoacetyl-L-aspartic acid, pyrazofurin, 6-azauridine, azaribine, and 3-deazauridine.
u. The sustained release formulation of clause q, wherein said antineoplastic drug is a pyrimidine nucleoside analog selected from the group consisting of arabinosyl cytosine, cyclocytidine, 5-aza-2'-deoxycytidine, arabinosyl 5-azacytosine, and 6-azacytidine.
v. The sustained release formulation of clause q, wherein said antineoplastic drug is selected from the group consisting of Cladribine, 6-mercaptopurine, pentostatin, 6-thioguanine, and fludarabin phosphate.
w. The sustained release formulation of any of clauses a-f, wherein the therapeutically active form of A is 5-fluorouracil.
x. The sustained release formulation of clauses a-f or i, wherein at least one of A or B is an anti-inflammatory agent.
y. The sustained release formulation of clause x, wherein said anti-inflammatory agent is a non-steroidal anti-inflammatory.
z. The sustained release formulation of clause y, wherein said anti-inflammatory agent is selected from the group consisting of diclofenac, fenoprofen, flurbiprofen, ibuprofen, ketoprofen, ketorolac, nahumstone, naproxen and piroxicam.
aa. The sustained release formulation of clause x, wherein anti-intlammatory agent is a glucocorticoid.
ab. The sustained release formulation of clause aa, wherein said glucocorticoid is selected from the group consisting of aclometasone, beclomethasone, betamethasone, budesonide, clobetasol, clobetasone, cortisone, desonide, desoximetasone, diflorosane, flumethasone, flunisolide, fluocinolone acetonide, fluocinolone, fluocortolone, fluprednidene, flurandrenolide, fluticasone, hydrocortisone, methylprednisolone aceponate,mometasone furdate, prednisolone, prednisone and rofleponide.
ac. The sustained release formulation of clause i, wherein the therapeutically active form of B is selected from fluocinolone acetonide, triamcinolone acetonide, diclofenac, and naproxen.
ad. The sustained release formulation of clause a, wherein the linkage L is hydrolyzed in bodily fluid.
ae. The sustained release formulation of clause a, wherein the linkage L includes one or more hydrolyzable groups selected from the group consisting of an ester, an amide, a carbamate, a carbonate, a cyclic ketal, a thioester, a thioamide, a thiocarbamate, a thiocarbonate, a xanthate and a phosphate ester.
af. The sustained release formulation of clause a, wherein the linkage L is enzymatically cleaved.
ag. The sustained release formulation of clause a, wherein the prodrug, in its linked form, has an ED₅₀ for producing said clinical response at least 10 times greater than the ED₅₀ of the therapeutically active form of A.
ah. The sustained release formulation of clause a, wherein the prodrug, in its linked form, has an ED₅₀ for producing said clinical response at least 1000 times greater than the ED₅₀ of the therapeutically active form of A.
ai. The sustained release formulation of clause a, wherein the therapeutically active form of A is at least 10 times more soluble in water relative to said prodrug.
aj. The sustained release formulation of clause ac, wherein the prodrug is selected from 5FU covalently bonded to fluocinolone acetonide, 5FU covalently bonded to naproxen, and 5FU covalently bonded to diclofenac.
ak. The sustained release formulation of clause i, wherein the prodrug is selected from ciprofloxacin-diclofenac (VI) and ciprofloxacin-naproxen. 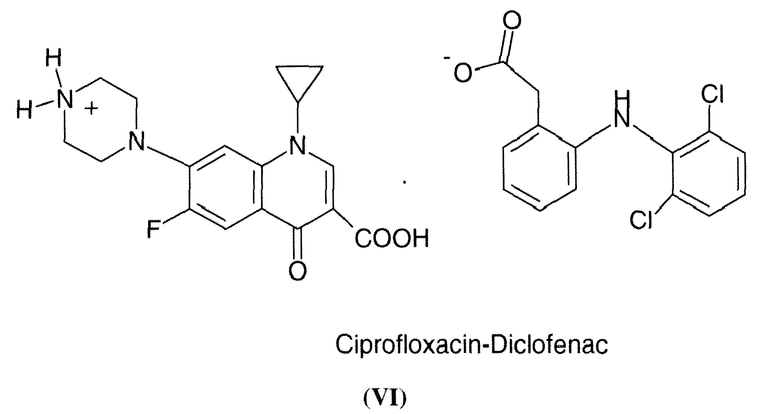
al. The sustained release formulation of any of clauses a-f, wherein the polymer is non-bioerodible.
am. The sustained release formulation of clause al, wherein the non-bioerodible polymer is selected from polyurethane, polysilicone, poly(ethylene-co-vinyl acetate), polyvinyl alcohol, and derivatives and copolymers thereof.
an. The sustained release formulation of any of clauses a-f, wherein the polymer is bioerodible.
ao. The sustained release formulation of clause an, wherein the bioerodible polymer is selected from polyanhydride, polylactic acid, polyglycolic acid, polyorthoester, polyalkylcyanoacrylate, and derivatives and copolymers thereof.
ap. The sustained release formulation of any of clauses a-f, wherein the polymer holds the prodrug in a particular anatomic position and prevents disintegration of the prodrug.
aq. The sustained release formulation of any of clauses a-f, wherein the polymer reduces interaction between the prodrug in the polymer and proteinaceous components in surrounding bathing fluid.
ar. The sustained release formulation of any of clauses a-f, wherein the system is adapted to be injected or implanted into a body.
as. A medical device comprising:
   (i) a substrate having a surface; and,
   (ii) a coating adhered to the surface, said coating comprising a polymer matrix having a low solubility prodrug dispersed therein, wherein said low solubility prodrug is represented by the general formula **A-L-B,** in which
      A represents a drug moiety having a therapeutically active form for producing a clinical response in a patient;
      L represents a covalent linker linking A and B to form a prodrug, said linker being cleaved under physiological conditions to generate said therapeutically active form of A; and
      B represents a moiety which, when linked to A, results in the prodrug having a lower solubility than the therapeutically active form of A.
at. The device of clause as, wherein the polymer matrix is essentially non-release rate limiting with respect to the rate of release of the therapeutically active form of A from the coating.
au. The device of clause as, wherein the substrate is a surgical implement selected from a screw, a plate, a washer, a suture, a prosthesis anchor, a tack, a staple, an electrical lead, a valve, and a membrane.
av. The device of clause as, selected from the group consisting of catheters, implantable vascular access ports, blood storage bags, blood tubing, central venous catheters, arterial catheters, vascular grafts, intraaortic balloon pumps, heart valves, cardiovascularsutures, artificial hearts, a pacemaker, ventricular assist pumps, extracorporeal devices, blood filters, hemodialysis units, hemoperfasion units, plasmapheresis units, and filters adapted for deployment in a blood vessel.
aw. The device of clause as, which is a vascular stent.
ax. The device of clause aw, which is an expandable stent, and said coating is flexible to accommodate compressed and expanded states of said expandable stent.
ay. The device of clause as, wherein the weight of the coating attributable to the prodrug is in the range of about 0.05 mg to about 50 mg of prodrug per cm² of the surface coated with said polymer matrix.
az. The device of clause as, wherein the coating has a thickness is in the range of 5 micrometers to 100 micrometers.
ba. The device of clause as, wherein prodrug is present in an amount between 5% and 70% by weight of the coating.
bb. A coated device combination, comprising a medical device for implantation within a patient's body, said medical device having one or more surfaces coated with a polymer formulation of any of clauses a-f in a manner that permits the coated surface to release the therapeutically active form of A over a period of time when implanted in the patient.
bc. The coated device of clause bb, wherein the device is an elongate radially expandable tubular stent having an interior luminal surface and an opposite exterior surface extending along a longitudinal stent axis.
bd. A stent having at least a portion which is insertable or implantable into the body of a patient, wherein the portion has a surface which is adapted for exposure to body tissue and wherein at least a part of the surface is covered with a coating for releasing at least one biologically active material, the coating comprising a polymer matrix having a low solubility prodrug dispersed therein, wherein said low solubility prodrug is represented by the general formula **A-L-B,** in which
   A represents a drug moiety having a therapeutically active form for producing a clinical response in a patient;
   L represents a covalent linker linking A and B to form a prodrug, said linker being cleaved under physiological conditions to generate said therapeutically active form of A; and
   B represents a moiety which, when linked to A, results in the prodrug having a lower solubility than the therapeutically active form of A.
be. An intraluminal medical device coated with a sustained release system comprising a biologically tolerated polymer and a low-solubility prodrug dispersed in the polymer, said device having an interior surface and an exterior surface; said device having said system applied to at least a part of the interior surface, the exterior surface, or both.
bf. A method for treating an intraluminal tissue of a patient, the method comprising the steps of:
   (a) providing a stent having an interior surface and an exterior surface, said stent having a coating on at least a part of the interior surface, the exterior surface, or both; said coating comprising a low-solubility pharmaceutical prodrug dissolved or dispersed in a biologicallytolerated polymer;
   (b) positioning the stent at an appropriate intraluminal tissue site; and
   (c) deploying the stent.
bg. A coating composition for use in delivering a medicament from the surface of a medical device positioned in vivo, the composition comprising a polymer matrix having a low solubility prodrug dispersed therein, wherein said low solubility prodrug is represented by the general formula **A-L-B,** in which
   A represents a drug moiety having a therapeutically active form for producing a clinical response in a patient;
   L represents a covalent linker linking A and B to form a prodrug, said linker being cleaved under physiological conditions to generate said therapeutically active form of A; and
   B represents a moiety which, when linked to A, results in the prodrug having a lower solubility than the therapeutically active form of A;
   which coating composition is provided in liquid or suspension form for application to the surface of said medical device by spraying and/or dipping the device in said composition.
bh. A coating composition for use in delivering a medicament from the surface of a medical device positioned in vivo, the composition comprising a polymer matrix having a low solubility prodrug dispersed therein, wherein said low solubility prodrug is represented by the general formula **A-L-B,** in which
   A represents a drug moiety having a therapeutically active form for producing a clinical response in a patient;
   L represents a covalent linker linking A and B to form a prodrug, said linker being cleaved under physiological conditions to generate said therapeutically active form of A;
   B represents a moiety which, when linked to A, results in the prodrug having a lower solubility than the therapeutically active form of A;
   which coating composition is provided in powdered form and, upon addition of a solvent, can reconstitute a liquid or suspension form for application to the surface of said medical device by spraying and/or dipping the device in said composition.
bi. An injectable composition for use in delivering a medicament to a patient, the composition comprising a polymer matrix having a low solubility prodrug dispersed therein, wherein said low solubility prodrug is represented by the general formula **A-L-B,** in which
   A represents a drug moiety having a therapeutically active form for producing a clinical response in a patient;
   L represents a covalent linker linking A and B to form a prodrug, said linker being cleaved under physiological conditions to generate said therapeutically active form of A;
   B represents a moiety which, when linked to A, results in the prodrug having a lower solubility than the therapeutically active form of A;
   which composition is provided in liquid or suspension form adapted for delivery by injection through a needle.
bj. A method of manufacturing a sustained release system, comprising admixing a polymer matrix and a therapeutically effective amount of a low solubility prodrug, wherein
   (i) said low solubility prodrug is represented by the general formula **A-L-B,** in which
      A represents a drug moiety having a therapeutically active form for producing a clinical response in a patient;
      L represents a covalent linker linking A and B to form a prodrug, said linker being cleaved under physiological conditions to generate said therapeutically active form of A;
      B represents a moiety which, when linked to A, results in the prodrug having a lower solubility than the therapeutically active form of A; and
   (ii) the polymer matrix is permeable to the therapeutically active form of A, and is essentially non-release rate limiting with respect to a rate of release of therapeutically active form of A from the polymer matrix.
bk. The method of clause bj, further comprising the step of applying the mixture of polymer matrix and prodrug to a surface of a surgical implement.
bl. A method for treating a mammalian organism to obtain a desired local or systemic physiological or pharmacological effect, comprising: administering a therapeutically effective amount of The sustained release formulation of any of clauses a-f to a mammal.
bm. A use of a sustained release system of any of clauses a-f in the manufacture of a medication for treating a patient with a sustained dosage regimen of the therapeutically active form of A.
bn. The sustained release formulation of clause e or f, wherein the therapeutically active form of A has a logP value at least 2 logP unit less than the logP value of the prodrug.

## Claims

1. A sustained release formulation, optionally adapted to be injected or implanted into a body, comprising
a) a polymer matrix, and
b) a codrug, dispersed in the polymer matrix, having a general formula of **A-L-B** in which
A represents a drug moiety having a therapeutically active form for producing a clinical response in a patient;
L represents a covalent linker linking A and B to form a codrug, said linker being cleaved under physiological conditions to generate said therapeutically active forms of A and B; and
B represents a drug moiety having a therapeutically active form generated upon cleavage of said linker L, which, when linked to A, results in the codrug having a lower solubility than the therapeutically active form of A,
wherein 1) the solubility of the therapeutically active form of A in water is greater than 1 mg/ml and the solubility of the codrug in water is less than 1 mg/ml, 2) when disposed in biological fluid, said sustained release formulation provides sustained release of the therapeutically active form of A for a period of at least 24 hours, and, over the period of release, the concentration of the codrug in fluid outside the polymer is less than 10% of the concentration of the therapeutically active form of A, and/or 3) the therapeutically active form of A has a logP value at least 1 logP unit less than the logP value of the codrug.

2. The sustained release formulation of claim 1 wherein said polymer matrix comprises a polymer optionally selected from polyurethane, polysilicone, poly(ethylene-co-vinyl acetate), polyvinyl alcohol, and derivatives and copolymers thereof, or a bioerodible polymer optionally selected from polyanhydride, polylactic acid, polyglycolic acid, polyorthoester, polyalkylcyanoacrylate, and derivatives and copolymers thereof.

3. The sustained release formulation of claim 1 or 2 wherein A is selected from immune response modifiers, anti-proliferatives, corticosteroids, angiostatic steroids, anti-parasitic drugs, anti-glaucoma drugs, antibiotics, anti-sense compounds, differentiation modulators, antiviral drugs, anticancer drugs, and non-steroidal anti-inflammatory drugs, such as 5-fluorouracil.

4. The sustained release formulation of anyone of claims 1 to 3 wherein L is hydrolyzable in bodily fluid or enzymatically cleavable, and optionally includes one or more hydrolyzable groups selected from an ester, an amide, a carbamate, a carbonate, a cyclic ketal, a thioester, a thioamide, a thiocarbamate, a thiocarbonate, a xanthate and a phosphate ester.

5. The sustained release formulation of anyone of claims 1 to 4 wherein B is selected from immune response modifiers, anti-proliferatives, corticosteroids, angiostatic steroids, anti-parasitic drugs, anti-glaucoma drugs, antibiotics, anti-sense compounds, differentiation modulators, antiviral drugs, anticancer drugs, and non-steroidal anti-inflammatory drugs, such as naproxen.

6. The sustained release formulation of anyone of claims 1 to 5, optionally wherein the polymer holds the codrug in a particular anatomic position and prevents disintegration of the codrug and/or reduces interaction between the codrug in the polymer and proteinaceous components in surrounding bathing fluid.

7. The sustained release formulation of claim 1 in the form of a medical device comprising:
(a) a substrate having a surface; and,
(b) a coating adhered to the surface, said coating comprising the sustained release formulation of claim 1;
wherein the polymer matrix is optionally essentially non-release rate limiting with respect to the rate of release of the therapeutically active form of A from the coating.

8. The sustained release formulation of claim 1 in the form of a stent having at least a portion which is insertable or implantable into the body of a patient, wherein the portion has a surface which is adapted for exposure to body tissue and wherein at least a part of the surface is covered with a coating for releasing at least one biologically active material, the coating comprising the sustained release formulation of claim 1.

9. A coating composition for use in delivering a medicament from the surface of a medical device positioned in vivo, the composition comprising the sustained release formulation of claim 1;
which coating composition is a) provided in liquid or suspension form for application to the surface of said medical device by spraying and/or dipping the device in said composition, or b) provided in powdered form and, upon addition of a solvent, reconstitutes a liquid or suspension form for application to the surface of said medical device by spraying and/or dipping the device in said composition.

10. An injectable composition for use in delivering a medicament to a patient, the composition comprising the sustained release formulation of claim 1;
which composition is provided in liquid or suspension form adapted for delivery by injection through a needle.

11. A method of manufacturing a sustained release system, comprising admixing a polymer matrix and a therapeutically effective amount of a low solubility codrug, wherein
(i) said low solubility codrug is represented by the general formula **A-L-B**, in which
A represents a drug moiety having a therapeutically active form for producing a clinical response in a patient;
L represents a covalent linker linking A and B to form a codrug, said linker being cleaved under physiological conditions to generate said therapeutically active form of A and a therapeutically active form of B; and
B represents a drug moiety having said therapeutically active form for producing a clinical response in a patient which, when linked to A, results in the codrug having a lower solubility than the therapeutically active form of A; and
(ii) the polymer matrix is permeable to the therapeutically active form of **A,** and is non-release rate limiting with respect to a rate of release of therapeutically active form of A from the polymer matrix.

12. The method of claim 11, further comprising the step of applying the mixture of polymer matrix and codrug to a surface of a surgical implement.

13. The sustained release formulation of claim 1 for use in the preparation of a medicament to administer a therapeutically effective amount of said formulation to obtain a desired local or systemic physiological or pharmacological effect.

14. A sustained release system of claim 1 for use in the manufacture of a medicament for treating a patient with a sustained dosage regimen of the therapeutically active form of A.
